# EUROPÄISCHE PATENTSCHRIFT

(11) **EP 0 710 657 B1**
(45) Veröffentlichungstag und Bekanntmachung des Hinweises auf die Patenterteilung: **26.08.1998**
(21) Anmeldenummer: 95116483.9
(22) Anmeldetag: 19.10.1995
(51) Int. Cl.: C07D 263/24, A61K 31/42

(54) **Adhäsionsrezeptor-Antagonisten**
Antagonists of adhesion receptors
Antagonistes des récepteurs d'adhésion

(30) Priorität: 02.11.1994 DE 4439110; 16.03.1995 DE 19509093
(43) Veröffentlichungstag der Anmeldung: 08.05.1996
(73) Patentinhaber: MERCK PATENT GmbH, 64293 Darmstadt (DE)
(72) Erfinder: Gante, Joachim, Prof., D-64291 Darmstadt (DE); Juraszyk, Horst, Dr., D-64342 Seeheim (DE); Raddatz, Peter, Dr., D-64342 Seeheim (DE); Wurziger, Hanns, Dr., D-64291 Darmstadt (DE); Bernotat-Danielowski, Sabine, Dr., D-61231 Bad Nauheim (DE); Melzer, Guido, Dr., D-65713 Hofheim/Ts (DE); Wiesner, Matthias, Dr., D-55128 Mainz (DE); Fittschen, Claus, Dr., D-64407 Fr.-Crumbach (DE)

(56) Entgegenhaltungen:
- EP-A- 0 086 403
- EP-A- 0 352 781
- EP-A- 0 511 031
- EP-A- 0 605 729
- GB-A- 834 968
- DISSERTATION VON BEATE DIEFENBACH, 1995, DARMSTADT;'UNTERSUCHUNGEN ZUR AFFINITÄTSMODULATION DER BETA3-INTEGRIN-LIGAND-WECHSELWIRKUNG'
- PSCHYREMBOL, 1990, SEITE 200,201

## Beschreibung

Die Erfindung betrifft neue Oxazolidinon-Derivate der Formel I 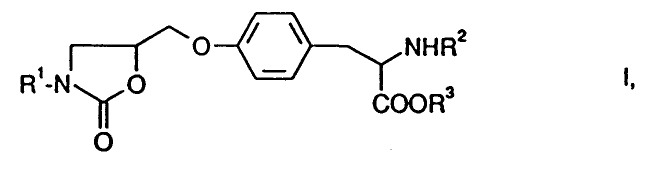 worin
- R¹: 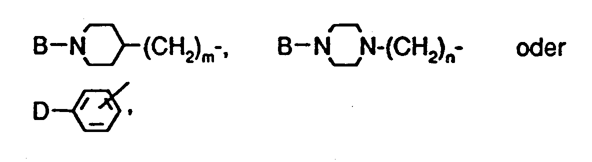
- R²: H, A, Ac, A-SO₂-, Ar-SO₂- oder eine konventionelle Aminoschutzgruppe,
- R³: H, A, Cycloalkyl mit 3 bis 7 C-Atomen, Ar oder Ar-(CH₂)ₖ-,
- A: Alkyl mit 1 bis 16 C-Atomen,
- B: H, A oder H₂N-C(=NH)-,
- D: H₂N-CH₂-, H₂N-C(=NH)- oder H₂N-C(=NH)-NH-CH₂, wobei die primären Aminogruppen auch mit konventionellen Aminoschutzgruppen versehen sein können,
- Ac: Alkanoyl mit 1 bis 10 C-Atomen oder Aroyl mit 7 bis 11 C-Atomen,
- Ar: unsubstituiertes oder durch A, Cl, Br, I, OA, OH, NO₂, CN, NH₂, NHA oder NA₂ mono- und/oder disubstituiertes Phenyl oder Benzyl,
- m: 0, 1, 2, 3 oder 4,
- n: 2, 3 oder 4 und
- k: 1, 2, 3 oder 4 bedeuten,
sowie deren physiologisch unbedenkliche Salze.

Ähnliche Verbindungen sind aus der EP-A1-0 381 033 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Vor allem wirken sie als Integrin-Inhibitoren, wobei sie insbesondere die Wechselwirkungen der β₃- oder β₅-Integrin-Rezeptoren mit Liganden hemmen. Besondere Wirksamkeit zeigen die Verbindungen im Falle der Integrine aᵥβ₃ aᵥβ₅ und a_{IIb}β₃. Diese Wirkung kann z.B. nach der Methode nachgewiesen werden, die von J.W. Smith et al. in J. Biol. Chem. 2265, 12267-12271 (1990) beschrieben wird. Insbesondere hemmen sie die Bindung von Fibrinogen, Fibronectin und des von-Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Angina pectoris, osteolytischen Erkrankungen, insbesondere Osteoporose, Antiangiogenese und Restenose nach Angioplastie, Ischämien, Entzündungen, Arteriosklerose und von akutem Nierenversagen verwendet werden. Ferner haben die Verbindungen einen Effekt auf Tumorzellen, indem sie deren Metastasierung hemmen. Somit können sie auch als Anti-Tumor-Mittel eingesetzt werden.

Es gibt Hinweise, daß Tumorzellen durch Mikrothrombi in die Gefäße gelangen und somit vor der Detektion durch die Zellen des Immunsystems geschützt sind. Ebenso wirken Mikrothrombi unterstützend auf die Bindung der Tumorzellen an die Gefäßwände. Da die Bildung der Mikrothrombi im Zusammenhang mit der Fibrinogen-Bindung zum Fibrinogen-Rezeptor (Glycoprotein IIb/IIIa) steht, gelten Fibrinogen-Bindungs-Inhibitoren ebenfalls als Metastase-Inhibitoren.

Die Verbindungen eignen sich zudem als antimikrobielle Wirkstoffe, die Infektionen, wie sie beispielsweise durch Bakterien, Pilze oder Hefen ausgelöst werden, verhindern können. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika. Antimikrobielle Aktivitäten der Verbindungen können z.B. nach der Methode von P. Valentin-Weigand et al., beschrieben in Infection and Immunity, 2851-2855 (1988), nachgewiesen werden.

Die anderen Eigenschaften der Verbindungen können nach Methoden nachgewiesen werden, die in der EP-A1-0 462 960 beschrieben sind. Die Hemmung der Fibrinbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen.

Gegenstand der Erfindung ist ferner ein Verfahren zur Herstellung einer Verbindung der angegebenen Formel I sowie von deren Salzen, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Verbindung der Formel II 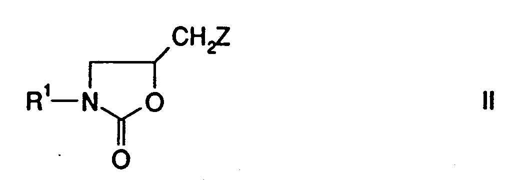 worin
   - Z: Cl, Br, I, OH oder eine reaktionsfähig veresterte OH-Gruppe
   bedeutet, und
   - R¹: die oben angegebene Bedeutung besitzt,
   mit einer Verbindung der Formel III 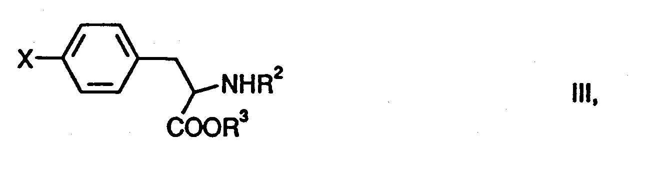 worin
   - R² und R³: die angegebenen Bedeutungen haben
   und
   - X: OH oder einen aus OH ableitbaren salzartigen Rest bedeutet, umsetzt, oder daß man
(c) eine Verbindung der Formel IV 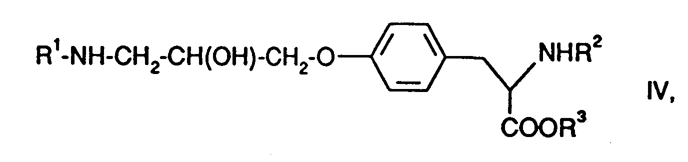 worin
   R¹, R² und R³ die angegebenen Bedeutungen haben,
   mit einem reaktionsfähigen Derivat der Kohlensäure umsetzt, oder daß man
(d) eine Verbindung der Formel V 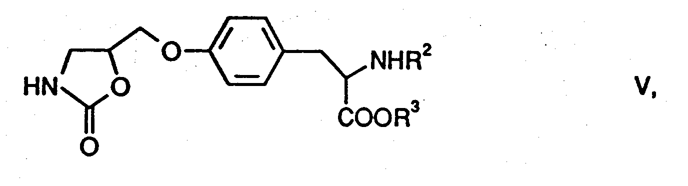 worin
   - R² und R³: die angegebenen Bedeutungen haben, mit einer Verbindung der Formel VI 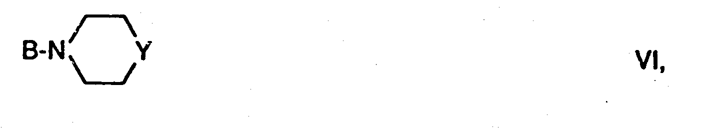
   worin
   - B: die angegebene Bedeutung hat
   und
   - Y: 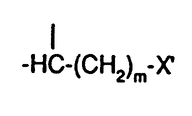 oder 〉N-(CH₂)ₙ-X',
   worin

   - m und n: die bereits angegebenen Bedeutungen haben und
   - X': Cl, Br, I oder eine andere, leicht nucleophil verdrängbare Abgangsgruppe ist, bedeutet,
   umsetzt,
(e) zur Herstellung einer Guanidinomethylverbindung der Formel I (R¹ = einfach durch H₂N-C(=NH)-NH-CH₂- substituierter Phenylrest) eine Aminoverbindung entsprechend der Formel I, die jedoch anstelle des Restes R¹ eine Aminomethylphenylgruppe enthält, mit einem amidinierenden Mittel behandelt, oder daß man
(f) einen Rest R³ in einen anderen Rest R³ umwandelt, indem man einen Ester der Formel I verseift, oder eine Carbonsäure der Formel I verestert, oder daß man
(g) (einen) Rest(e) R¹ und/oder R² in (einen) andere(n) Rest(e) R¹ und/oder R² umwandelt, und/oder daß man
(h) eine Verbindung der Formel I durch Behandeln mit einer Säure oder Base in eines ihrer Salze überführt.

Die Verbindungen der Formel I besitzen mindestens ein chirales Zentrum und können daher in mehreren enantiomeren Formen auftreten. Alle diese Formen (z.B. D- und L-Formen) und deren Gemische (z.B. die DL-Formen) sind in der Formel I eingeschlossen.

Vor- und nachstehend haben die Reste bzw. Parameter A, B, D, X, Y, Z, R¹ bis R³, Ac, Ar, k, m und n die bei den Formeln I bis VI angegebenen Bedeutungen, falls nicht ausdrücklich etwas anderes angegeben ist. Falls mehrere gleich bezeichnete Gruppen im Molekül vorhanden sind, können sie unabhängig voneinander verschiedene Definitionen annehmen.

In den vorstehenden Formeln hat die Gruppe A 1-6, vorzugsweise 1, 2, 3 oder 4 C-Atome. Im einzelnen bedeutet A vorzugsweise Methyl, Ethyl, Propyl, Isopropyl, Butyl, Isobutyl, sek-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl.

R¹ ist vorzugsweise ein in 4-Stellung, aber auch in 2- oder 3-Stellung wie angegeben substituierter Phenylrest, im einzelnen bevorzugt 2-, 3- oder (insbesondere) 4-Aminomethylphenyl, 2-, 3- oder (insbesondere) 4-Amidinophenyl, 2-, 3- oder 4-Guanidinomethylphenyl, wobei in allen Fällen die primären Aminogruppen auch mit konventionellen Aminoschutzgruppen versehen sein können. Ferner ist R¹ auch bevorzugt ein 4-Piperidyl-, 4-Piperidylmethyl-, 4-Piperidylethyl-, 1-Amidino-4-piperidyl-, 1-Amidino-4-piperidylmethyl-, 4-Piperazinylethyl-, 4-Piperazinylpropyl-, 4-Piperazinylbutyl-, 1-Amidino-4-piperazinylethyl- oder ein 1-Amidino-4-piperazinylpropyl-Rest.

R² steht vorzugsweise für Wasserstoff, Methyl, Ethyl, Methyl-, Ethyl-, n-Propyl- oder n-Butylsulfonyl, Toluolsulfonyl oder eine konventionelle Aminoschutzgruppe.

R³ ist bevorzugt Wasserstoff, Methyl oder Ethyl.

Ar ist vorzugsweise unsubstituiertes Phenyl oder 4-Methylphenyl, Ac ist vorzugsweise Alkanoyl mit 1-6 C-Atomen wie Formyl, Acetyl, Propionyl, Butyryl, Isobutyryl, Valeryl oder Caproyl, ferner Benzoyl, Toluyl, 1- oder 2-Naphthoyl oder Phenylacetyl.

Die Parameter k und m sind vorzugsweise 0 oder 1. Der Parameter n ist vorzugsweise 2 oder 3.

Unter den Verbindungen der Formel I sind diejenigen bevorzugt, in denen mindestens einer der angegebenen Reste, Gruppen und/oder Parameter eine der angegebenen bevorzugten Bedeutungen hat. Einige Gruppen von bevorzugten Verbindungen sind diejenigen der Formeln Ia bis If, die der Formel I entsprechen, worin jedoch
in Ia
   - R¹: Amidinophenyl bedeutet;
in Ib
   - R¹: Amidinophenyl und
   - R²: Wasserstoff bedeutet;
Ic
   - R¹: Aminomethylphenyl und
   - R³: Wasserstoff oder Methyl bedeutet;
Id
   - R¹: Amidinophenyl und
   - R³: Wasserstoff oder Methyl bedeutet;
Ie
   - R¹: Piperidylmethyl und Piperidylethyl und
   - R²: Wasserstoff oder A-SO₂- bedeutet;
If
   - R¹: 1-Amidinopiperidylmethyl und
   - R²: Wasserstoff oder A-SO₂- bedeuet;
Ig
   - R¹: Piperazinoethyl oder -propyl und
   - R²: Wasserstoff oder A-SO₂- bedeutet;
Ih
   - R¹: 1-Amidinopiperazinoethyl oder -propyl und
   - R²: Wasserstoff oder A-SO₂- bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A1-0 381 033, EP-A1-0 462 960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, insbesondere solche, die an Stelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch an Stelle einer Gruppe -COOH eine Gruppe -COOR'' tragen, worin R'' eine Hydroxyschutzgruppe bedeutet.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe an Stelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, Sulfoxide wie Dimethylsulfoxid (DMSO), ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können bevorzugt auch durch Reaktion einer Verbindung der Formel II mit einem Phenolderivat der Formel III erhalten werden. Dabei bedient man sich zweckmäßig der an sich bekannten Methoden zur Herstellung von Ethern.

Die Fluchtgruppe Z bedeutet vorzugsweise Cl, Br, I, C₁-C₆-Alkylsulfonyloxy wie Methan- oder Ethansulfonyloxy oder C₆-C₁₀-Arylsulfonyloxy wie Benzol-, p-Toluol- oder 1- oder 2-Naphthalinsulfonyloxy.

Die Reaktion gelingt vorzugsweise in Gegenwart einer zusätzlichen Base, z.B. eines Alkali- oder Erdalkalimetall-hydroxids oder carbonats wie Natrium-, Kalium- oder Calciumhydroxid, Natrium-, Kalium- oder Calciumcarbonat, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Falls die Fluchtgruppe E von I verschieden ist, empfiehlt sich ein Zusatz eines Iodids wie Kaliumiodid.

Die Ausgangsstoffe der Formel II sind in der Regel neu. Sie können z.B. hergestellt werden durch Reaktion eines substituierten Anilins der Formel R¹-NH₂ mit einer Verbindung der Formel R⁵CH₂-CHR⁶-CH₂OH (worin R⁵ Z, R⁶ OH oder R⁵ und R⁶ zusammen auch O bedeuten) zu einer Verbindung der Formel R¹-NH-CH₂-CHR⁸-CH₂OH (worin R⁸ OH bedeutet), Reaktion mit einem Derivat der Kohlensäure wie Diethylcarbonat zu 3-R¹-5-hydroxymethyl-2-oxazolidinonen und gegebenenfalls Umwandlung der Hydroxymethylgruppe in eine CH₂Z-Gruppe, z.B. mit SOCl₂, SOBr₂, Methansulfonylchlorid oder p-Toluolsulfonylchlorid. Die Verbindungen der Formel III sind in der Regel bekannt oder in Analogie zu bekannten Verbindungen aus geeigneten Phenolderivaten oder aus Phenol herstellbar.

Verbindungen der Formel I können ferner erhalten werden durch Reaktion einer Verbindung der Formel IV (oder eines reaktionsfähigen Derivats davon) mit einem reaktiven Derivat der Kohlensäure.

Als Kohlensäurederivate eignen sich insbesondere Dialkylcarbonate wie Diethylcarbonat, ferner auch Chlorameisensäurealkylester wie Ethyl-chlorformiat. Bevorzugt dient das Kohlensäurederivat, das zweckmäßig im Überschuß eingesetzt wird, auch als Lösungs- bzw. Suspensionsmittel. Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kalium-tert.-butylat. Man arbeitet zweckmäßig bei Reaktionstemperaturen zwischen 0 und 150°, vorzugsweise zwischen 70 und 120°.

Die Ausgangsstoffe der Formel IV sind in der Regel neu. Sie sind z.B. erhältlich durch Funktionalisierung der oben genannten Verbindungen der Formel R¹-NH-CH₂-CH(OH)-CH₂OH zu Verbindungen der Formel R¹-NH-CH₂-CH(OH)-CH₂-Z und Reaktion mit Verbindungen der Formel III.

Zur Herstellung von Verbindungen der Formel I, worin R¹ eine Guanidinophenylgruppe bedeutet, kann man eine entsprechende Aminophenylverbindung mit einem amidinierenden Mittel behandeln. Als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Weiterhin ist es möglich, in einer Verbindung der Formel I einen Rest R³ in einen anderen Rest R³ umzuwandeln, indem man einen Ester der Formel I verseift oder eine Carbonsäure der Formel I verestert.

Zur Veresterung kann man eine Säure der Formel I (R³ = H) mit einem Überschuß eines Alkohols der Formel R³-OH (R³ = A oder Benzyl) behandeln, zweckmäßig in Gegenwart einer starken Säure wie Salzsäure oder Schwefelsäure bei Temperaturen zwischen 0 und 100, vorzugsweise 20 und 50°.

Umgekehrt kann ein Ester der Formel I (R³ = A oder Benzyl), in die entsprechende Säure der Formel I (R³ = H) umgewandelt werden, zweckmäßig durch Solvolyse oder Hydrogenolyse nach einer der oben angegebenen Methoden, z.B. mit NaOH oder KOH in Wasser-Dioxan bei Temperaturen zwischen 0 und 40°, vorzugsweise 10 und 30°.

Ferner ist es möglich, daß man einen Rest R¹ und/oder R² in einen anderen Rest R¹ und/oder R² umwandelt.

Insbesondere kann man primäre oder sekundäre Aminogruppen alkylieren, acylieren, amidinieren oder mit konventionellen Aminoschutzgruppen oder Alkyl- oder Arylsulfonylgruppen versehen bzw. in umgekehrter Weise durch Entfernung dieser Gruppen freisetzen.

Zur Herstellung eines Amidins der Formel I (R¹ = Amidinophenyl) kann man an ein Nitril der Formel I (R¹ = Cyanphenyl) Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃I, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCl in den entsprechenden Imidoester umwandelt und diesen mit Ammoniak behandelt, oder c) das Nitril mit Lithiumbis-(trimethylsilyl)-amid umsetzt und das Produkt anschließend hydrolysiert.

Analog sind die entsprechenden N-Hydroxy-amidine der Formel I (R¹ = durch HO-NH-C(=NH)-substituiertes Phenyl) aus den Nitrilen erhältlich, wenn man nach a) oder b), aber mit Hydroxylamin an Stelle von Ammoniak arbeitet. Diese Produkte können dann weiter derivatisiert werden, indem man sie z.B. mit Wasserstoffgas reduziert.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel I (R³ = H) durch Umsetzung mit entsprechenden Basen in ihre Metall- oder Ammoniumsalze umzuwandeln, z.B. ihre Natrium-, Kalium- oder Calciumsalze.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/ Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosieiungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzyl alkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensattresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459 256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Ge schlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, filtriert über eine Ionenaustauschersäule, trocknet die organische Phase über Natriumsulfat, dampft ein, lyophilisiert gegebenenfalls und reinigt durch Chromatographie an Kieselgel und/oder Kristallisation. In den nachfolgenden Beispielen bedeutet "4-piperidylethyl" stets "2-(4-piperidyl)-ethyl", "4-piperidylpropyl" stets "3-(4-piperidyl)-propyl" und "4-piperidylbutyl" stets "4-(4-piperidyl)-butyl". Ebenso bedeutet "4-piperazinylethyl" stets "2-(4-piperazinyl)-ethyl", "4-piperazinylpropyl" "3-(4-piperazinyl)-propyl" und "4-piperazinylbutyl" "4-(4-piperazinyl)-butyl". Eingeschlossen sind hierbei auch die mit Schutzgruppen versehenen Derivate, z.B. die BOC-geschützten Verbindungen.

### Beispiel 1

Zu einer Lösung von 1,9 g 2-N-BOC-Amino-3-(4-hydroxy-phenyl)-propansäuremethylester ("A") [erhältlich aus Tyrosin durch Veresterung mit Methanol und Einführung der BOC-Schutzgruppe] in 20 ml Dimethylformamid (DMF) gibt man 1 Äquivalent NaH und rührt 30 Min. bei Raumtemperatur. Danach fügt man 1,8g 3-p-N-BOC-Amidino-phenyl-5-methansulfonyloxy-methyl-oxazolidin-2-on [erhältlich durch Reaktion von p-Aminobenzonitril mit 2,3-Epoxypropan-1-ol zu p-(N-2,3-Dihydroxypropyl-amino)-benzonitril, Umsetzung mit Diethylcarbonat in Gegenwart von K-tert.-butylat zu 3-p-Cyanphenyl-5-hydroxymethyl-oxazolidin-2-on, Umsetzung mit H₂S, Methyliodid und Ammonium-acetat zum Amidin, Einführung einer BOC-Schutzgruppe am Amidin und anschließende Veresterung mit Methansulfonylchlorid], gelöst in 10 ml DMF, hinzu und rührt erneut 15 Min. bei Raumtemperatur. Nach Entfernung des Lösungsmittels und üblicher Aufarbeitung erhält man das 3-p-BOC-Amidinophenyl-5-[p-(2-methoxycarbonyl-2-N-BOC-amino-ethyl)-phenoxy]-methyloxazolidin-2-on.

Analog erhält man durch Umsetzung von "A"
mit 3-(4-N-BOC-Piperidyl)-5-methansulfonyloxy-methyl-oxazolidin-2-on das
3-(4-N-BOC-Piperidyl)-5-[p-(2-methoxycarbonyl-2-N-BOC-amino-ethyl)-phenoxy]-methyl-oxazolidin-2-on;
mit 3-(4-N-BOC-Piperidylmethyl)-5-methansulfonyloxy-methyl-oxazolidin-2-on das
3-(4-N-BOC-Piperidylmethyl)-5-[p-(2-methoxycarbonyl-2-N-BOC-amino-ethyl)-phenoxy]-methyl-oxazolidin-2-on;
mit 3-(4-N-BOC-Piperidylethyl)-5-methansulfonyloxy-methyl-oxazolidin-2-on das
3-(4-N-BOC-Piperidylethyl)-5-[p-(2-methoxycarbonyl-2-N-BOC-amino-ethyl)-phenoxy]-methyl-oxazolidin-2-on;
mit 3-(4-N-BOC-Piperidylpropyl)-5-methansulfonyloxy-methyl-oxazolidin-2-on das
3-(4-N-BOC-Piperidylpropyl)-5-[p-(2-methoxycarbonyl-2-N-BOC-amino-ethyl)-phenoxy]-methyl-oxazolidin-2-on;
mit 3-(4-N-BOC-Piperidylbutyl)-5-methansulfonyloxy-methyl-oxazolidin-2-on das
3-(4-N-BOC-Piperidylbutyl)-5-[p-(2-methoxycarbonyl-2-N-BOC-amino-ethyl)-phenoxy]-methyl-oxazolidin-2-on;
mit 3-(1-N-BOC-Amidino-4-piperidyl)-5-methansulfonyloxy-methyl-oxazolidin-2-on das
3-(1-N-BOC-Amidino-4-piperidyl)-5-[p-(2-methoxycarbonyl-2-N-BOC-amino-ethyl)-phenoxy]-methyl-oxazolidin-2-on;
mit 3-(1-N-BOC-Amidino-4-piperidylmethyl)-5-methansulfonyloxy-methyl-oxazolidin-2-on das
3-(1-N-BOC-Amidino-4-piperidylmethyl)-5-[p-(2-methoxycarbonyl-2-N-BOC-amino-ethyl)-phenoxy]-methyl-oxazolidin-2-on;
mit 3-(1-N-BOC-Amidino-4-piperidylethyl)-5-methansulfonyloxy-methyl-oxazolidin-2-on das
3-(1-N-BOC-Amidino-4-piperidylethyl)-5-[p-(2-methoxycarbonyl-2-N-BOC-amino-ethyl)-phenoxy]-methyl-oxazolidin-2-on;
mit 3-(1-N-BOC-Amidino-4-piperidylpropyl)-5-methansulfonyloxy-methyl-oxazolidin-2-on das
3-(1-N-BOC-Amidino-4-piperidylpropyl)-5-[p-(2-methoxycarbonyl-2-N-BOC-amino-ethyl)-phenoxy]-methyl-oxazolidin-2-on;
mit 3-(1-N-BOC-Amidino-4-piperidylbutyl)-5-methansulfonyloxy-methyl-oxazolidin-2-on das
3-(1-N-BOC-Amidino-4-piperidylbutyl)-5-[p-(2-methoxycarbonyl-2-N-BOC-amino-ethyl)-phenoxy]-methyl-oxazolidin-2-on;
mit 3-(1-Isopropyl-4-piperidyl)-5-methansulfonyloxy-methyl-oxazolidin-2-on das
3-(1-Isopropyl-4-piperidyl)-5-[p-(2-methoxycarbonyl-2-N-BOC-amino-ethyl)-phenoxy]-methyl-oxazolidin-2-on;
mit 3-(1-Methyl-4-piperidylmethyl)-5-methansulfonyloxy-methyl-oxazolidin-2-on das
3-(1-Methyl-4-piperidylmethyl)-5-[p-(2-methoxycarbonyl-2-N-BOC-amino-ethyl)-phenoxy]-methyl-oxazolidin-2-on;
mit 3-(4-N-BOC-Piperazinylethyl)-5-methansulfonyloxy-methyl-oxazolidin-2-on das
3-(4-N-BOC-Piperazinylethyl)-5-[p-(2-methoxycarbonyl-2-N-BOC-amino-ethyl)-phenoxy]-methyl-oxazolidin-2-on;
mit 3-(4-N-BOC-Piperazinylpropyl)-5-methansulfonyloxy-methyl-oxazolidin-2-on das
3-(4-N-BOC-Piperazinylpropyl)-5-[p-(2-methoxycarbonyl-2-N-BOC-amino-ethyl)-phenoxy]-methyl-oxazolidin-2-on;
mit 3-(4-N-BOC-Piperazinylbutyl)-5-methansulfonyloxy-methyl-oxazolidin-2-on das
3-(4-N-BOC-Piperazinylbutyl)-5-[p-(2-methoxycarbonyl-2-N-BOC-amino-ethyl)-phenoxy]-methyl-oxazolidin-2-on;
mit 3-(1-N-BOC-Amidino-4-piperazinylethyl)-5-methansulfonyloxy-methyl-oxazolidin-2-on das
3-(1-N-BOC-Amidino-4-pipenazinylethyl)-5-[p-(2-methoxycarbonyl-2-N-BOC-amino-ethyl)-phenoxy]-methyl-oxazolidin-2-on;
mit 3-(1-N-BOC-Amidino-4-piperazinylpropyl)-5-methansulfonyloxy-methyl-oxazolidin-2-on das
3-(1-N-BOC-Amidino-4-piperazinylpropyl)-5-[p-(2-methoxycarbonyl-2-N-BOC-amino-ethyl)-phenoxy]-methyl-oxazolidin-2-on;
mit 3-(1-N-BOC-Amidino-4-piperazinylbutyl)-5-methansulfonyloxy-methyl-oxazolidin-2-on das
3-(1-N-BOC-Amidino-4-piperazinylbutyl)-5-[p-(2-methoxycarbonyl-2-N-BOC-amino-ethyl)-phenoxyl-methyl-oxazolidin-2-on.

### Beispiel 2

Eine Lösung von 0,9 g 3-p-Cyan-phenyl-5-[p-(2-methoxycarbonyl-2-N-BOC-amino-ethyl)-phenoxy]-methyl-oxazolidin-2-on [erhältlich nach Bsp. 1 durch Umsetzung von 2-N-BOC-Amino-3-(4-hydroxy-phenyl)-propan säuremethylester mit 3-p-Cyan-phenyl-5-methansulfonyloxy-methyl-oxazolidin-2-on] in 40 ml 10%iger methanolischer NH₃-Lösung wird an 0,6 g Raney-Ni bei Raumtemperatur und 1bar bis zum Ende der H₂-Aufnahme hydriert. Nach Filtrieren und Eindampfen erhält man nach üblicher Aufarbeitung 3-p-Aminomethyl-phenyl-5-[p-(2-methoxycarbonyl-2-N-BOC-amino-ethyl)-phenoxy]-methyl-oxazolidin-2-on.

Analog erhält man
aus 3-m-Cyan-phenyl-5-[p-(2-methoxycarbonyl-2-N-BOC-amino-ethyl)-phenoxy]-methyl-oxazolidin-2-on das
3-m-Aminomethyl-phenyl-5-[p-(2-methoxycarbonyl-2-N-BOC-amino-ethyl)-phenoxy]-methyl-oxazolidin-2-on;
aus 3-m-Cyan-phenyl-5-[p-(2-methoxycarbonyl-2-N-butylsulfonyl-amino-ethyl)-phenoxy]-methyl-oxazolidin-2-on das
3-m-Aminomethyl-phenyl-5-[p-(2-methoxycarbonyl-2-N-butylsulfonyl-amino-ethyl)-phenoxy]-methyl-oxazolidin-2-on;
aus 3-p-Cyan-phenyl-5-[p-(2-methoxycarbonyl-2-N-butylsulfonyl-amino-ethyl)-phenoxy]-methyl-oxazolidin-2-on das
3-p-Aminomethyl-phenyl-5-[p-(2-methoxycarbonyl-2-N-butylsulfonyl-amino-ethyl)-phenoxy]-methyl-oxazolidin-2-on;
aus 3-o-Cyan-phenyl-5-[p-(2-methoxycarbonyl-2-N-butylsulfonyl-amino-ethyl)-phenoxy]-methyl-oxazolidin-2-on das
3-o-Aminomethyl-phenyl-5-[p-(2-methoxycarbonyl-2-N-butylsulfonyl-amino-ethyl)-phenoxy]-methyl-oxazolidin-2-on;
aus 3-o-Cyan-phenyl-5-[p-(2-methoxycarbonyl-2-N-BOC-aminoethyl)-phenoxy]-methyl-oxazolidin-2-on das
3-o-Aminomethyl-phenyl-5-[p-(2-methoxycarbonyl-2-N-BOC-amino-ethyl)-phenoxy]-methyl-oxazolidin-2-on.

### Beispiel 3

Analog Beispiel 1 erhält man ausgehend von 2-N-Butylsulfonyl-amino-3-(4-hydroxy-phenyl)-propan-säuremethylester ("B") [erhältlich aus Tyrosin durch Veresterung mit Methanol und Umsetzung mit Butylsulfonylchlorid] durch Umsetzung mit 3-(1-N-BOC-Amidino-4-piperidyl)-5-methansulfonyloxy-methyl-oxazolidin-2-on [erhältlich durch Reaktion von 1-N-BOC-Amidino-4-amino-piperidin mit 2,3-Epoxypropan-1-ol zu 1-N-BOC-Amidino-4-(N-2,3-dihydroxy-propyl-amino)-piperidin, Umsetzung mit Diethylcarbonat in Gegenwart von K-tert.-butylat zu 3-(1-N-BOC-Amidino-4-piperidyl)-5-hydroxy-methyl-oxazolidin-2-on und anschließende Veresterung mit Methansulfonylchlorid] das 3-(1-N-BOC-Amidino-4-piperidyl)-5-[p-(2-methoxycarbonyl-2-N-butylsulfonyl-amino-ethyl)-phenoxy]-methyl-oxazolidin-2-on.

Analog erhält man durch Umsetzung von "B"
mit 3-(4-N-BOC-Piperidyl)-5-methansulfonyloxy-methyl-oxazolidin-2-on das
3-(4-N-BOC-Piperidyl)-5-[p-(2-methoxycarbonyl-2-N-butylsulfonyl-amino-ethyl)-phenoxy]-methyl-oxazolidin-2-on;
mit 3-(4-N-BOC-Piperidylmethyl)-5-methansulfonyloxy-methyl-oxazolidin-2-on das
3-(4-N-BOC-Piperidylmethyl)-5-[p-(2-methoxycarbonyl-2-N-butylsulfonyl-amino-ethyl)-phenoxy]-methyl-oxazolidin-2-on;
mit 3-(4-N-BOC-Piperidylethyl)-5-methansulfonyloxy-methyl-oxazolidin-2-on das
3-(4-N-BOC-Pipendylethyl)-5-[p-(2-methoxycarbonyl-2-N-butylsulfonyl-amino-ethyl)-phenoxy]-methyl-oxazolidin-2-on;
mit 3-(4-N-BOC-Piperidylpropyl)-5-methansulfonyloxy-methyl-oxazolidin-2-on das
3-(4-N-BOC-Piperidylpropyl)-5-[p-(2-methoxycarbonyl-2-N-butylsulfonyl-amino-ethyl)-phenoxy]-methyl-oxazolidin-2-on;
mit 3-(4-N-BOC-Piperidylbutyl)-5-methansulfonyloxy-methyl-oxazolidin-2-on das
3-(4-N-BOC-Piperidylbutyl)-5-[p-(2-methoxycarbonyl-2-N-butylsulfonyl-amino-ethyl)-phenoxy]-methyloxazolidin-2-on;
mit 3-(1-Ethyl-4-piperidyl)-5-methansulfonyloxy-methyl-oxazolidin-2-on das 3-(1-Ethyl-4-piperidyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperidylmethyl)-5-methansulfonyloxy-methyl-oxazolidin-2-on das
3-(1-N-BOC-Amidino-4-piperidylmethyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperidylethyl)-5-methansulfonyloxy-methyl-oxazolidin-2-on das
3-(1-N-BOC-Amidino-4-piperidylethyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperidylpropyl)-5-methansulfonyloxy-methyl-oxazolidin-2-on das
3-(1-N-BOC-Amidino-4-piperidylpropyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperidylbutyl)-5-methansulfonyloxy-methyl-oxazolidin-2-on das
3-(1-N-BOC-Amidino-4-piperidylbutyl)-5-[p-(2-methoxycarbonyl-2-N-butylsulfonyl-amino-ethyl)-phenoxy]-methyl-oxazolidin-2-on;
mit 3-(1-Isopropyl-4-piperidyl)-5-methansulfonyloxy-methyl-oxazolidin-2-on das
3-(1-Isopropyl-4-piperidyl)-5-[p-(2-methoxycarbonyl-2-N-butylsulfonyl-amino-ethyl)-phenoxy]-methyl-oxazolidin-2-on;
mit 3-(1-Methyl-4-piperidylmethyl)-5-methansulfonyloxy-methyl-oxazolidin-2-on das
3-(1-Methyl-4-piperidylmethyl)-5-[p-(2-methoxycarbonyl-2-N-butylsulfonyl-amino-ethyl)-phenoxy]-methyl-oxazolidin-2-on;
mit 3-(4-N-BOC-Piperazinylethyl)-5-methansulfonyloxy-methyl-oxazolidin-2-on das
3-(4-N-BOC-Piperazinylethyl)-5-[p-(2-methoxycarbonyl-2-N-butylsulfonyl-amino-ethyl)-phenoxy]-methyl-oxazolidin-2-on;
mit 3-(4-N-BOC-Piperazinylpropyl)-5-methansulfonyloxy-methyl-oxazolidin-2-on das
3-(4-N-BOC-Piperazinylpropyl)-5-[p-(2-methoxycarbonyl-2-N-butylsulfonyl-amino-ethyl)-phenoxy]-methyl-oxazolidin-2-on;
mit 3-(4-N-BOC-Piperazinylbutyl)-5-methansulfonyloxy-methyl-oxazolidin-2-on das
3-(4-N-BOC-Piperazinylbutyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperazinylethyl)-5-methansulfonyloxy-methyl-oxazolidin-2-on das
3-(1-N-BOC-Amidino-4-piperazinylethyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperazinylpropyl)-5-methansulfonyloxy-methyl-oxazolidin-2-on das
3-(1-N-BOC-Amidino-4-piperazinylpropyl)-5-[p-(2-methoxycarbonyl-2-N-butylsulfonyl-amino-ethyl)-phenoxy]-methyl-oxazolidin-2-on;
mit 3-(1-N-BOC-Amidino-4-piperazinylbutyl)-5-methansulfonyloxy-methyl-oxazolidin-2-on das
3-(1-N-BOC-Amidino-4-piperazinylbutyl)-5-[p-(2-methoxycarbonyl-2-N-butylsulfonyl-amino-ethyl)-phenoxy]-methyl-oxazolidin-2-on.

### Beispiel 4

1,2 g 3-p-Amidino-phenyl-5-[p-(2-methoxycarbonyl-2-N-BOC-amino-ethyl)-phenoxy]-methyl-oxazolidin-2-on [erhältlich nach Bsp. 1] werden in 60 ml Methanol suspendiert, mit 4 ml 2 N NaOH-Lösung versetzt und 4 Stunden bei Raumtemperatur gerührt. Nach Entfernung des Lösungsmittels wird der Rückstand in Wasser aufgenommen, der pH-Wert durch Zugabe von verdünnter HCl auf 3 eingestellt und über einen sauren Ionenaustauscher filtriert. Das Filtrat wird über MgSO₄ getrocknet. Nach Entfernung des Lösungsmittels und zusätzlicher Lyophilisation erhält man 3-p-Amidinophenyl-5-[p-(2-carboxy-2-N-BOC-amino-ethyl)-phenoxy]-methyl-oxazolidin-2-on.

Analog erhält man durch Verseifung der Produkte aus Beispiel 1
3-(4-N-BOC-Piperidyl)-5-[p-(2-carboxy-2-N-BOC-amino-ethyl)-phenoxy]-methyl-oxazolidin-2-on;
3-(4-N-BOC-Piperidylmethyl)-5-[p-(2-carboxy-2-N-BOC-amino-ethyl)-phenoxy]-methyl-oxazolidin-2-on;
3-(4-N-BOC-Piperidylethyl)-5-[p-(2-carboxy-2-N-BOC-amino-ethyl)-phenoxy]-methyl-oxazolidin-2-on;
3-(4-N-BOC-Piperidylpropyl)-5-[p-(2-carboxy-2-N-BOC-amino-ethyl)-phenoxy]-methyl-oxazolidin-2-on;
3-(4-N-BOC-Piperidylbutyl)-5-[p-(2-carboxy-2-N-BOC-amino-ethyl)-phenoxy]-methyl-oxazolidin-2-on;
3-(1-N-BOC-Amidino-4-piperidyl)-5-[p-(2-carboxy-2-N-BOC-amino-ethyl)-phenoxy]-methyl-oxazolidin-2-on;
3-(1-N-BOC-Amidino-4-piperidylmethyl)-5-[p-(2-carboxy-2-N-BOC-amino-ethyl)-phenoxy]-methyl-oxazolidin-2-on;
3-(1-N-BOC-Amidino-4-piperidylethyl)-5-[p-(2-carboxy-2-N-BOC-amino-ethyl)-phenoxy]-methyl-oxazolidin-2-on;
3-(1-N-BOC-Amidino-4-piperidylpropyl)-5-[p-(2-carboxy-2-N-BOC-amino-ethyl)-phenoxy]-methyl-oxazolidin-2-on;
3-(1-N-BOC-Amidino-4-piperidylbutyl)-5-[p-(2-carboxy-2-N-BOC-amino-ethyl)-phenoxy]-methyl-oxazolidin-2-on;
3-(1-Isopropyl-4-piperidyl)-5-[p-(2-carboxy-2-N-BOC-amino-ethyl)-phenoxy]-methyl- oxazolidin-2-on;
3-(1-Methyl-4-piperidylmethyl)-5-[p-(2-carboxy-2-N-BOC-amino-ethyl)-phenoxy]-methyl-oxazolidin-2-on;
3-(4-N-BOC-Piperazinylethyl)-5-[p-(2-carboxy-2-N-BOC-amino-ethyl)-phenoxy]-methyl-oxazolidin-2-on;
3-(4-N-BOC-Piperazinylpropyl)-5-[p-(2-carboxy-2-N-BOC-amino-ethyl)-phenoxy]-methyl-oxazolidin-2-on;
3-(4-N-BOC-Piperazinylbutyl)-5-[p-(2-carboxy-2-N-BOC-amino-ethyl)-phenoxy]-methyl-oxazolidin-2-on;
3-(1-N-BOC-Amidino-4-piperazinylethyl)-5-[p-(2-carboxy-2-N-BOC-amino-ethyl)-phenoxy]-methyl-oxazolidin-2-on;
3-(1-N-BOC-Amidino-4-piperazinylpropyl)-5-[p-(2-carboxy-2-N-BOC-amino-ethyl)-phenoxy]-methyl-oxazolidin-2-on;
3-(1-N-BOC-Amidino-4-piperazinylbutyl)-5-[p-(2-carboxy-2-N-BOC-amino-ethyl)-phenoxy]-methyl-oxazolidin-2-on.

### Beispiel 5

0,6 g 3-p-N-BOC-Amidino-phenyl-5-[p-(2-carboxy-2-N-BOC-amino-ethyl)-phenoxy]-methyl-oxazolidin-2-on [erhältlich nach Bsp. 4] werden in 40 ml 2 N HCl-Lösung auf Dioxanbasis suspendiert und 3 Stunden bei Raumtemperatur gerührt. Nach Entfernung des Lösungsmittels und üblicher Aufarbeitung erhält man 3-p-Amidino-phenyl-5-[p-(2-carboxy-2-amino-ethyl)-phenoxy]-methyl-oxazolidin-2-on, Dihydrochlorid, F. 165° (Zers.).

Analog erhält man nach Entfernung der BOC-Schutzgruppe der Produkte aus Beispiel 4
3-(4-Piperidyl)-5-[p-(2-carboxy-2-amino-ethyl)-phenoxy]-methyl-oxazolidin-2-on, Dihydrochlorid;
3-(4-Piperidylmethyl)-5-[p-(2-carboxy-2-amino-ethyl)-phenoxy]-methyl-oxazolidin-2-on, Dihydrochlorid;
3-(4-Piperidylethyl)-5-[p-(2-carboxy-2-amino-ethyl)-phenoxy]-methyl-oxazolidin-2-on, Dihydrochlorid;
3-(4-Piperidylpropyl)-5-[p-(2-carboxy-2-amino-ethyl)-phenoxy]-methyl-oxazolidin-2-on, Dihydrochlorid;
3-(4-Piperidylbutyl)-5-[p-(2-carboxy-2-amino-ethyl)-phenoxy]-methyl-oxazolidin-2-on, Dihydrochlorid;
3-(1-Amidino-4-piperidyl)-5-[p-(2-carboxy-2-amino-ethyl)-phenoxy]-methyl-oxazolidin-2-on, Dihydrochlorid;
3-(1-Amidino-4-piperidylmethyl)-5-[p-(2-carboxy-amino-ethyl)-phenoxy]-methyl-oxazolidin-2-on, Dihydrochlorid;
3-(1-Amidino-4-piperidylethyl)-5-[p-(2-carboxy-2-amino-ethyl)-phenoxy]-methyl-oxazolidin-2-on, Dihydrochlorid;
3-(1-Amidino-4-piperidylpropyl)-5-[p-(2-carboxy-2-amino-ethyl)-phenoxy]-methyl-oxazolidin-2-on, Dihydrochlorid;
3-(1-Amidino-4-piperidylbutyl)-5-[p-(2-carboxy-2-amino-ethyl)-phenoxy]-methyl-oxazolidin-2-on, Dihydrochlorid;
3-(1-Isopropyl-4-piperidyl)-5-[p-(2-carboxy-2-amino-ethyl)-phenoxy]-methyl-oxazolidin-2-on, Dihydrochlorid;
3-(1-Methyl-4-piperidylmethyl)-5-[p-(2-carboxy-2-amino-ethyl)-phenoxy]-methyl-oxazolidin-2-on, Dihydrochlorid;
3-(4-Piperazinylethyl)-5-[p-(2-carboxy-2-amino-ethyl)-phenoxy]-methyl-oxazolidin-2-on, Dihydrochlorid;
3-(4-Piperazinylpropyl)-5-[p-(2-carboxy-2-amino-ethyl)-phenoxy]-methyl-oxazolidin-2-on, Dihydrochlorid;
3-(4-Piperazinylbutyl)-5-[p-(2-carboxy-2-amino-ethyl)-phenoxy]-methyl-oxazolidin-2-on, Dihydrochlorid;
3-(1-Amidino-4-piperazinylethyl)-5-[p-(2-carboxy-2-amino-ethyl)-phenoxy]-methyl-oxazolidin-2-on, Dihydrochlorid;
3-(1-Amidino-4-piperazinylpropyl)-5-[p-(2-carboxy-2-amino-ethyl)-phenoxy]-methyl-oxazolidin-2-on, Dihydrochlorid;
3-(1-Amidino-4-piperazinylbutyl)-5-[p-(2-carboxy-2-amino-ethyl)-phenoxy]-methyl-oxazolidin-2-on, Dihydrochlorid.

### Beispiel 6

0,6 g 3-p-N-BOC-Amidino-phenyl-5-[p-(2-methoxycarbonyl-2-N-BOC-amino-ethyl)-phenoxy]-methyl-oxazolidin-2-on [erhältlich nach Bsp. 1] werden in 40 ml 2 N HCl-Lösung auf Dioxanbasis suspendiert und 3 Stunden bei Raumtemperatur gerührt. Nach Entfernung des Lösungsmittels und üblicher Aufarbeitung erhält man 3-p-Amidino-phenyl-5-[p-(2-methoxycarbonyl-2-amino-ethyl)-phenoxy]-methyl-oxazolidin-2-on, Dihydrochlorid, F. 132-133°.

Analog erhält man nach Entfernung der BOC-Schutzgruppe der Produkte aus Beispiel 1
3-(4-Piperidyl)-5-[p-(2-methoxycarbonyl-2-amino-ethyl)-phenoxy]-methyl-oxazolidin-2-on, Dihydrochlorid;
3-(4-Piperidylmethyl)-5-[p-(2-methoxycarbonyl-2-amino-ethyl)-phenoxy]-methyl-oxazolidin-2-on, Dihydrochlorid;
3-(4-Piperidylethyl)-5-[p-(2-methoxycarbonyl-2-amino-ethyl)-phenoxy]-methyl-oxazolidin-2-on, Dihydrochlorid;
3-(4-Piperidylpropyl)-5-[p-(2-methoxycarbonyl-2-amino-ethyl)-phenoxy]-methyl-oxazolidin-2-on, Dihydrochlorid;
3-(4-Piperidylbutyl)-5-[p-(2-methoxycarbonyl-2-amino-ethyl)-phenoxy]-methyl-oxazolidin-2-on, Dihydrochlorid;
3-(1-Amidino-4-piperidyl)-5-[p-(2-methoxycarbonyl-2-amino-ethyl)-phenoxy]-methyl-oxazolidin-2-on, Dihydrochlorid;
3-(1-Amidino-4-piperidylmethyl)-5-[p-(2-methoxycarbonyl-amino-ethyl)-phenoxy]-methyl-oxazolidin-2-on, Dihydrochlorid;
3-(1-Amidino-4-piperidylethyl)-5-[p-(2-methoxycarbonyl-2-amino-ethyl)-phenoxy]-methyl-oxazolidin-2-on, Dihydrochlorid;
3-(1-Amidino-4-piperidylpropyl)-5-[p-(2-methoxycarbonyl-2-amino-ethyl)-phenoxy]-methyl-oxazolidin-2-on, Dihydrochlorid;
3-(1-Amidino-4-piperidylbutyl)-5-[p-(2-methoxycarbonyl-2-amino-ethyl)-phenoxy]-methyl-oxazolidin-2-on, Dihydrochlorid;
3-(1-Isopropyl-4-piperidyl)-5-[p-(2-methoxycarbonyl-2-amino-ethyl)-phenoxy]-methyl-oxazolidin-2-on, Dihydrochlorid;
3-(1-Methyl-4-piperidylmethyl)-5-[p-(2-methoxycarbonyl-2-amino-ethyl)-phenoxy]-methyl-oxazolidin-2-on, Dihydrochlorid;
3-(4-Piperazinylethyl)-5-[p-(2-methoxycarbonyl-2-amino-ethyl)-phenoxy]-methyl-oxazolidin-2-on, Dihydrochlorid;
3-(4-Piperazinylpropyl)-5-[p-(2-methoxycarbonyl-2-amino-ethyl)-phenoxy]-methyl-oxazolidin-2-on, Dihydrochlorid;
3-(4-Piperazinylbutyl)-5-[p-(2-methoxycarbonyl-2-amino-ethyl)-phenoxy]-methyl-oxazolidin-2-on, Dihydrochlorid;
3-(1-Amidino-4-piperazinylethyl)-5-[p-(2-methoxycarbonyl-2-amino-ethyl)-phenoxy]-methyl-oxazolidin-2-on, Dihydrochlorid;
3-(1-Amidino-4-piperazinylpropyl)-5-[p-(2-methoxycarbonyl-2-amino-ethyl)-phenoxy]-methyl-oxazolidin-2-on, Dihydrochlorid;
3-(1-Amidino-4-piperazinylbutyl)-5-[p-(2-methoxycarbonyl-2-amino-ethyl)-phenoxy]-methyl-oxazolidin-2-on, Dihydrochlorid.

### Beispiel 7

Analog Beispiel 4 erhält man ausgehend von 3-(1-N-BOC-Amidino-4-piperidyl)-5-[p-(2-methoxy-carbonyl-2-N-butylsulfonyl-amino-ethyl)-phenoxy]-methyl-oxazolidin-2-on [erhältlich nach Bsp. 3] durch Verseifung das 3-(1-N-BOC-Amidino-4-piperidyl)-5-[p-(2-carboxy-2-N-butylsulfonyl-amino-ethyl)-phenoxy]-methyl-oxazolidin-2-on.

Analog erhält man durch Verseifung
von 3-(4-N-BOC-Piperidyl)-5-[p-(2-methoxycarbonyl-2-N-butylsulfonyl-amino-ethyl)-phenoxy]-methyl-oxazolidin-2-on:
3-(4-N-BOC-Piperidyl)-5-[p-(2-carboxy-2-N-butylsulfonyl-amino-ethyl)-phenoxy]-methyl-oxazolidin-2-on;
von 3-(4-N-BOC-Piperidylmethyl)-5-[p-(2-methoxycarbonyl-2-N-butyl-sulfonyl-amino-ethyl)-phenoxy]-methyl-oxazolidin-2-on:
3-(4-N-BOC-Piperidylmethyl)-5-[p-(2-carboxy-2-N-butylsulfonyl-amino-ethyl)-phenoxy]-methyl-oxazolidin-2-on;
von 3-(4-N-BOC-Piperidylethyl)-5-[p-(2-methoxycarbonyl-2-N-butyl-sulfonyl-amino-ethyl)-phenoxy]-methyl-oxazolidin-2-on:
3-(4-N-BOC-Piperidylethyl)-5-[p-(2-carboxy-2-N-butylsulfonyl-amino-ethyl)-phenoxy]-methyl-oxazolidin-2-on;
von 3-(4-N-BOC-Piperidylpropyl)-5-[p-(2-methoxycarbonyl-2-N-butyl-sulfonyl-amino-ethyl)-phenoxy]-methyl-oxazolidin-2-on:
3-(4-N-BOC-Piperidylpropyl)-5-[p-(2-carboxy-2-N-butylsulfonyl-amino-ethyl)-phenoxy]-methyl-oxazolidin-2-on;
von 3-(4-N-BOC-Piperidylbutyl)-5-[p-(2-methoxycarbonyl-2-N-butyl-sulfonyl-amino-ethyl)-phenoxy]-methyl-oxazolidin-2-on:
3-(4-N-BOC-Piperidylbutyl)-5-[p-(2-carboxy-2-N-butylsulfonyl-amino-ethyl)-phenoxy]-methyl-oxazolidin-2-on;
von 3-(1-Ethyl-4-piperidyl)-5-[p-(2-methoxycarbonyl-2-N-butylsulfonyl-amino-ethyl)-phenoxy]-methyl-oxazolidin-2-on:
3-(1-Ethyl-4-piperidyl)-5-[p-(2-carboxy-2-N-butylsulfonyl-amino-ethyl)-phenoxy]-methyl-oxazolidin-2-on;
von 3-(1-N-BOC-Amidino-4-piperidylmethyl)-5-[p-(2-methoxycarbonyl-2-N-butylsulfonyl-amino-ethyl)-phenoxy]-methyl-oxazolidin-2-on:
3-(1-N-BOC-Amidino-4-piperidylmethyl)-5-[p-(2-carboxy-2-N-butylsulfonyl-amino-ethyl)-phenoxy]-methyl-oxazolidin-2-on;
von 3-(1-N-BOC-Amidino-4-piperidylethyl)-5-[p-(2-methoxycarbonyl-2-N-butylsulfonyl-amino-ethyl)-phenoxy]-methyl-oxazolidin-2-on:
3-(1-N-BOC-Amidino-4-piperidylethyl)-5-[p-(2-carboxy-2-N-butylsulfonyl-amino-ethyl)-phenoxy]-methyl-oxazolidin-2-on;
von 3-(1-N-BOC-Amidino-4-piperidylpropyl)-5-[p-(2-methoxycarbonyl-2-N-butylsulfonyl-amino-ethyl)-phenoxy]-methyl-oxazolidin-2-on:
3-(1-N-BOC-Amidino-4-piperidylpropyl)-5-[p-(2-carboxy-2-N-butylsulfonyl-amino-ethyl)-phenoxy]-methyl-oxazolidin-2-on;
von 3-(1-N-BOC-Amidino-4-piperidylbutyl)-5-[p-(2-methoxycarbonyl-2-N-butylsulfonyl-amino-ethyl)-phenoxy]-methyl-oxazolidin-2-on:
3-(1-N-BOC-Amidino-4-piperidylbutyl)-5-[p-(2-carboxy-2-N-butylsulfonyl-amino-ethyl)-phenoxy]-methyl-oxazolidin-2-on;
von 3-(1-Isopropyl-4-piperidyl)-5-[p-(2-methoxycarbonyl-2-N-butylsulfonyl-amino-ethyl)-phenoxy]-methyl-oxazolidin-2-on:
3-(1-Isopropyl-4-piperidyl)-5-[p-(2-carboxy-2-N-butylsulfonyl-amino-ethyl)-phenoxy]-methyl-oxazolidin-2-on;
von 3-(1-Methyl-4-piperidylmethyl)-5-[p-(2-methoxycarbonyl-2-N-butyl-sulfonyl-amino-ethyl)-phenoxy]-methyl-oxazolidin-2-on:
3-(1-Methyl-4-piperidylmethyl)-5-[p-(2-carboxy-2-N-butylsulfonyl-amino-ethyl)-phenoxy]-methyl-oxazolidin-2-on;
von 3-(4-N-BOC-Piperazinylethyl)-5-[p-(2-methoxycarbonyl-2-N-butyl-sulfonyl-amino-ethyl)-phenoxy]-methyl-oxazolidin-2-on:
3-(4-N-BOC-Piperazinylethyl)-5-[p-(2-carboxy-2-N-bufylsulfonyl-amino-ethyl)-phenoxy]-methyl-oxazolidin-2-on;
von 3-(4-N-BOC-Piperazinylpropyl)-5-[p-(2-methoxycarbonyl-2-N-butylsulfonyl-amino-ethyl)-phenoxy]-methyl-oxazolidin-2-on:
3-(4-N-BOC-Piperazinylpropyl)-5-[p-(2-carboxy-2-N-butylsulfonyl-amino-ethyl)-phenoxy]-methyl-oxazolidin-2-on;
von 3-(4-N-BOC-Piperazinylbutyl)-5-[p-(2-methoxycarbonyl-2-N-butylsulfonyl-amino-ethyl)-phenoxy]-methyl-oxazolidin-2-on:
3-(4-N-BOC-Piperazinylbutyl)-5-[p-(2-carboxy-2-N-butylsulfonyl-amino-ethyl)-phenoxy]-methyl-oxazolidin-2-on;
von 3-(1-N-BOC-Amidino-4-piperazinylethyl)-5-[p-(2-methoxycarbonyl-2-N-butylsulfonyl-amino-ethyl)-phenoxy]-methyl-oxazolidin-2-on:
3-(1-N-BOC-Amidino-4-piperazinylethyl)-5-[p-(2-carboxy-2-N-butylsulfonyl-amino-ethyl)-phenoxy]-methyl-oxazolidin-2-on;
von 3-(1-N-BOC-Amidino-4-piperazinylpropyl)-5-[p-(2-methoxycarbonyl-2-N-butylsulfonyl-amino-ethyl)-phenoxy]-methyl-oxazolidin-2-on:
3-(1-N-BOC-Amidino-4-piperazinylpropyl)-5-[p-(2-carboxy-2-N-butylsulfonyl-amino-ethyl)-phenoxy]-methyl-oxazolidin-2-on;
von 3-(1-N-BOC-Amidino-4-piperazinylbutyl)-5-[p-(2-methoxycarbonyl-2-N-butylsulfonyl-amino-ethyl)-phenoxy]-methyl-oxazolidin-2-on:
3-(1-N-BOC-Amidino-4-piperazinylbutyl)-5-[p-(2-carboxy-2-N-butylsulfonyl-amino-ethyl)-phenoxy]-methyl-oxazolidin-2-on.

### Beispiel 8

Zu einer Lösung von 0,6 g 3-p-Amidino-phenyl-5-[p-(2-methoxycarbonyl-2-N-BOC-amino-ethyl)-phenoxy]-methyl-oxazolidin-2-on in 20 ml THF fügt man 20 ml 20%ige NaOH-Lösung hinzu und rührt 24 Std. bei Raumtemperatur. Man erhält nach Entfernung des Lösungsmittels und Gefriertrocknung 3-p-Amidino-phenyl-5-[p-(carboxy-2-N-BOC-amino-ethyl)-phenoxy]-methyl-oxazolidin-2-on-Na-Salz, F. 120-121°.

Analog erhält man
3-p-Aminomethyl-phenyl-5-[p-(carboxy-2-N-BOC-amino-ethyl)-phenoxy]-methyl-oxazolidin-2-on-Na-Salz;
3-p-Amidinophenyl-5-[p-(carboxy-2-N-butylsulfonyl-amino-ethyl)-phenoxy]-methyl-oxazolidin-2-on-Na-Salz;
3-p-Aminomethyl-phenyl-5-[p-(carboxy-2-N-butylsulfonyl-amino-ethyl)-phenoxy]-methyl-oxazolidin-2-on-Na-Salz.

### Beispiel 9

Eine Lösung von 0,2 g 1-Amidino-3,5-dimethylpyrazol-nitrat in 17 ml Dioxan und 5 ml Wasser wird mit 0,17 ml Ethyldiisopropylamin versetzt und 15 Min. gerührt. Anschließend gibt man 0,4 g 3-p-Aminomethyl-phenyl-5-[p-(2-methoxycarbonyl-2-N-BOC-amino-ethyl)-phenoxy]-methyl-oxazolidin-2-on hinzu, kocht das Gemisch 30 Std., dampft ein und arbeitet wie üblich auf. Man erhält 3-p-Guanidinomethyl-phenyl-5-[p-(2-methoxy-carbonyl-2-N-BOC-amino-ethyl)-phenoxy]-methyl-oxazolidin-2-on.

Analog erhält man
aus 3-m-Aminomethylphenyl-5-[p-(2-methoxycarbonyl-2-N-BOC-amino-ethyl)-phenoxy]-methyl-oxazolidin-2-on das
3-m-Guanidinomethyl-phenyl-5-[p-(2-methoxycarbonyl-2-N-BOC-amino-ethyl)-phenoxy]-methyl-oxazolidin-2-on;
aus 3-m-Aminomethyl-phenyl-5-[p-(2-methoxycarbonyl-2-N-butylsulfonyl-amino-ethyl)-phenoxy]-methyl-oxazolidin-2-on das
3-m-Guanidinomethyl-phenyl-5-[p-(2-methoxycarbonyl-2-N-butyl-sulfonyl-amino-ethyl)-phenoxy]-methyl-oxazolidin-2-on;
aus 3-p-Aminomethyl-phenyl-5-[p-(2-methoxycarbonyl-2-N-bulylsulfonyl-amino-ethyl)-phenoxy]-methyl-oxazolidin-2-on das
3-p-Guanidinomethyl-phenyl-5-[p-(2-methoxycarbonyl-2-N-butyl-sulfonyl-amino-ethyl)-phenoxy]-methyl-oxazolidin-2-on;
aus 3-o-Aminomethyl-phenyl-5-[p-(2-methoxycarbonyl-2-N-butylsulfonyl-amino-ethyl)-phenoxy]-methyl-oxazolidin-2-on das
3-o-Guanidinomethyl-phenyl-5-[p-(2-methoxycarbonyl-2-N-butyl-sulfonyl-amino-ethyl)-phenoxy]-methyl-oxazolidin-2-on;
aus 3-o-Aminomethyl-phenyl-5-[p-(2-methoxycarbonyl-2-N-BOC-amino-ethyl)-phenoxy]-methyl-oxazolidin-2-on das
3-o-Guanidinomethyl-phenyl-5-[p-(2-methoxycarbonyl-2-N-BOC-amino-ethyl)-phenoxy]-methyl-oxazolidin-2-on.

### Beispiel 10

Man leitet in eine Lösung von 1,2 g 3-p-Cyan-phenyl-5-[p-(2-methoxy-carbonyl-2-N-butylsulfonyl-amino-ethyl)-phenoxy]-methyl-oxazolidin-2-on [erhältlich nach Bsp. 1 durch Umsetzung von 2-N-Butylsulfonyl-amino-3-(4-hydroxy-phenyl)-propansäuremethylester mit 3-p-Cyan-phenyl-5-methansulfonyloxy-methyl-oxazolidin-2-on] in 50 ml Pyridin und 7 ml Triethylamin bei -10° H₂S-Gas bis zur Sättigung ein. Anschließend rührt man 14 Std. bei Raumtemperatur, dampft ein, löst den Rückstand in 50 ml Aceton und versetzt mit 9 ml Methyliodid. Nachdem man erneut 6 Std. gerührt hat, filtriert man ab, wäscht den Rückstand mit 5 ml Aceton, löst denselben in 30 ml Methanol, gibt 4,6 g Ammoniumacetat hinzu und rührt 24 Std. bei Raumtemperatur. Nach üblicher Aufarbeitung erhält man 3-p-Amidino-phenyl-5-[p-(2-methoxycarbonyl-2-N-butylsulfonyl-amino-ethyl)-phenoxy]-methyl-oxazolidin-2-on, F. 162-163°.

Analog erhält man
aus 3-m-Cyan-phenyl-5-[p-(2-methoxycarbonyl-2-N-butylsulfonyl-amino-ethyl)-phenoxy]-methyl-oxazolidin-2-on das
3-m-Amidino-phenyl-5-[p-(2-methoxycarbonyl-2-N-butylsulfonyl-amino-ethyl)-phenoxy]-methyl-oxazolidin-2-on;
aus 3-p-Cyan-phenyl-5-[p-(2-methoxycarbonyl-2-N-BOC-amino-ethyl)-phenoxy]-methyl-oxazolidin-2-on das
3-p-Amidino-phenyl-5-[p-(2-methoxycarbonyl-2-N-BOC-amino-ethyl)-phenoxy]-methyl-oxazolidin-2-on, F. 159-160°;
aus 3-p-Cyan-phenyl-5-[p-(2-methoxycarbonyl-2-N-p-toluolsulfonyl-amino-ethyl)-phenoxy]-methyl-oxazolidin-2-on das
3-p-Amidino-phenyl-5-[p-(2-methoxycarbonyl-2-N-p-toluolsulfonyl-amino-ethyl)-phenoxy]-methyl-oxazolidin-2-on;
aus 3-m-Cyan-phenyl-5-[p-(2-methoxycarbonyl-2-N-p-toluolsulfonyl-amino-ethyl)-phenoxy]-methyl-oxazolidin-2-on das
3-m-Amidino-phenyl-5-[p-(2-methoxycarbonyl-2-N-p-toluolsulfonyl-amino-ethyl)-phenoxy]-methyl-oxazolidin-2-on.

### Beispiel 11

Analog Beispiel 4 erhält man ausgehend von 3-p-Amidino-phenyl-5-[p-(2-methoxycarbonyl-2-N-butylsulfonyl-amino-ethyl)-phenoxy]-methyl-oxazolidin-2-on [erhältlich nach Bsp. 10] durch Verseifung das 3-p-Amidino-phenyl-5-[p-(2-carboxy-2-N-butylsulfonyl-amino-ethyl)-phenoxy]-methyl-oxazolidin-2-on, F. 236-237°.

Analog erhält man durch Verseifung
von 3-m-Amidino-phenyl-5-[p-(2-methoxycarbonyl-2-N-butylsulfonyl-amino-ethyl)-phenoxy]-methyl-oxazolidin-2-on das
3-m-Amidino-phenyl-5-[p-(2-carboxy-2-N-butylsulfonyl-amino-ethyl)-phenoxy]-methyl-oxazolidin-2-on;
von 3-p-Amidino-phenyl-5-[p-(2-methoxycarbonyl-2-N-p-toluolsulfonyl-amino-ethyl)-phenoxy]-methyl-oxazolidin-2-on das
3-p-Amidino-phenyl-5-[p-(2-carboxy-2-N-p-toluolsulfonyl-amino-ethyl)-phenoxy]-methyl-oxazolidin-2-on;
von 3-m-Amidino-phenyl-5-[p-(2-methoxycarbonyl-2-N-p-toluolsulfonyl-amino-ethyl)-phenoxy]-methyl-oxazolidin-2-on das
3-m-Amidino-phenyl-5-[p-(2-carboxy-2-N-p-toluolsulfonyl-amino-ethyl)-phenoxy]-methyl-oxazolidin-2-on.

### Beispiel 12

1,37 g 3-p-(5-Oxo-1,2,4-oxadiazolin-3-yl)-phenyl-5-[p-(2-methoxycarbonyl-2-N-ethyl-amino-ethyl)-phenoxy]-methyl-oxazolidin-2-on [erhältlich nach Bsp.-1 durch Umsetzung von 2-N-Ethyl-amino-3-(4-hydroxy-phenyl)-propansäuremethylester mit 3-p-(5-Oxo-1,2,4-oxadiazolin-3-yl)-phenyl-5-methan-sulfonylmethyl-oxazolidin-2-on], werden in 50 ml Methanol gelöst und an Raney-Nickel hydriert. Anschließend wird die Reaktionsmischung filtriert und das Filtrat im Vakuum eingeengt. Das erhaltene Produkt wird mit 20 ml Essigsäureethylester in der Wärme behandelt und das Produkt nach Abkühlen abgesaugt. Man erhält 3-p-Amidinophenyl-5-[p-(2-methoxycarbonyl-2-N-ethyl-amino-ethyl)-phenoxy]-methyl-oxazolidin-2-on.

Analog erhält man durch reduktive Spaltung der 5-Oxo-1,2,4-oxadiazolin-Gruppe
aus 3-p-(5-Oxo-1,2,4-oxadiazolin-3-yl)-phenyl-5-[p-(2-methoxycarbonyl-2-N-isopropyl-amino-ethyl)-phenoxy]-methyl-oxazolidin-2-on das
3-p-Amidinophenyl-5-[p-(2-methoxycarbonyl-2-N-isopropyl-amino-ethyl)-phenoxy]-methyl-oxazolidin-2-on;
aus 3-p-(5-Oxo-1,2,4-oxadiazolin-3-yl)-phenyl-5-[p-(2-methoxycarbonyl-2-N-acetyl-amino-ethyl)-phenoxy]-methyl-oxazolidin-2-on das
3-p-Amidinophenyl-5-[p-(2-methoxycarbonyl-2-N-acetyl-amino-ethyl)-phenoxy]-methyl-oxazolidin-2-on;
aus 3-p-(5-Oxo-1,2,4-oxadiazolin-3-yl)-phenyl-5-[p-(2-methoxycarbonyl-2-N-propionyl-amino-ethyl)-phenoxy]-methyl-oxazolidin-2-on das
3-p-Amidinophenyl-5-[p-(2-methoxycarbonyl-2-N-propionyl-amino-ethyl)-phenoxy]-methyl-oxazolidin-2-on;
aus 3-p-(5-Oxo-1,2,4-oxadiazolin-3-yl)-phenyl-5-[p-(2-methoxycarbonyl-2-N-BOC-amino-ethyl)-phenoxy]-methyl-oxazolidin-2-on das
3-p-Amidinophenyl-5-[p-(2-methoxycarbonyl-2-N-BOC-amino-ethyl)-phenoxy]-methyl-oxazolidin-2-on;
aus 3-p-(5-Oxo-1,2,4-oxadiazolin-3-yl)-phenyl-5-[p-(2-methoxycarbonyl-2-N-tert.-butyl-amino-ethyl)-phenoxy]-methyl-oxazolidin-2-on das
3-p-Amidinophenyl-5-[p-(2-methoxycarbonyl-2-N-tert.-butyl-amino-ethyl)-phenoxy]-methyl-oxazolidin-2-on.

### Beispiel 13

Analog Beispiel 4 erhält man durch Verseifung der entsprechenden Ester aus Beispiel 12 die folgenden Carbonsäuren:
3-p-Amidinophenyl-5-[p-(2-carboxy-2-N-ethyl-amino-ethyl)-phenoxy]-methyl-oxazolidin-2-on;
3-p-Amidinophenyl-5-[p-(2-carboxy-2-N-isopropyl-amino-ethyl)-phenoxy]-methyl-oxazolidin-2-on;
3-p-Amidinophenyl-5-[p-(2-carboxy-2-N-acetyl-amino-ethyl)-phenoxy]-methyl-oxazolidin-2-on;
3-p-Amidinophenyl-5-[p-(2-carboxy-2-N-propionyl-amino-ethyl)-phenoxy]-methyl-oxazolidin-2-on;
3-p-Amidinophenyl-5-[p-(2-carboxy-2-N-BOC-amino-ethyl)-phenoxy]-methyl-oxazolidin-2-on;
3-p-Amidinophenyl-5-[p-(2-carboxy-2-N-tert.-butyl-amino-ethyl)-phenoxy]-methyl-oxazolidin-2-on.

### Beispiel 14

Analog Beispiel 4 erhält man durch Verseifung der entsprechenden Ester aus Beispiel 9 die folgenden Carbonsäuren:
3-p-Guanidinomethyl-phenyl-5-[p-(2-carboxy-2-N-BOC-amino-ethyl)-phenoxy]-methyl-oxazolidin-2-on;
3-m-Guanidinomethyl-phenyl-5-[p-(2-carboxy-2-N-BOC-amino-ethyl)-phenoxy]-methyl-oxazolidin-2-on;
3-m-Guanidinomethyl-phenyl-5-[p-(2-carboxy-2-N-butylsulfonyl-amino-ethyl)-phenoxy]-methyl-oxazolidin-2-on;
3-p-Guanidinomethyl-phenyl-5-[p-(2-carboxy-2-N-butylsulfonyl-amino-ethyl)-phenoxy]-methyl-oxazolidin-2-on;
3-o-Guanidinomethyl-phenyl-5-[p-(2-carboxy-2-N-butylsulfonyl-amino-ethyl)-phenoxy]-methyl-oxazolidin-2-on;
3-o-Guanidinomethyl-phenyl-5-[p-(2-carboxy-2-N-BOC-amino-ethyl)-phenoxy]-methyl-oxazolidin-2-on.

### Beispiel 15

Analog Beispiel 5 erhält man durch Abspaltung der BOC-Schutzgruppe ausgehend von den Produkten aus Beispiel 14 die folgenden Verbindungen:
3-p-Guanidinomethyl-phenyl-5-[p-(2-carboxy-2-amino-ethyl)-phenoxy]-methyl-oxazolidin-2-on;
3-m-Guanidinomethyl-phenyl-5-[p-(2-carboxy-2-amino-ethyl)-phenoxy]-methyl-oxazolidin-2-on;
3-o-Guanidinomethyl-phenyl-5-[p-(2-carboxy-2-amino-ethyl)-phenoxy]-methyl-oxazolidin-2-on.

### Beispiel 16

0,5 g 3-p-(5-Oxo-1,2,4-oxadiazolin-3-yl)-phenyl-5-[p-(2-benzyloxycarbonyl-2-amino-ethyl)-phenoxy]-methyl-oxazolidin-2-on [erhältlich durch Umsetzung von 2-N-BOC-Amino-3-(4-hydroxy-phenyl)-propansäure-benzylester, welches man aus Tyrosin durch Einführung der BOC-Schutzgruppe und Veresterung mit Benzylalkohol erhält, mit 3-p-(5-Oxo-1,2,4-oxadiazolin-3-yl)-phenyl-5-methan-sulfonylmethyl-oxazolidin-2-on zu 3-p-(5-Oxo-1,2,4-oxadiazolin-3-yl)-phenyl-5-[p-(2-benzyloxycarbonyl-2-N-BOC-amino-ethyl)-phenoxy]-methyl-oxazolidin-2-on und anschließende Behandlung mit Trifluoressigsäure] werden in 10 ml Dichlormethan gelöst und mit 1 Äquivalent Propansulfonsäurechlorid und 2 Äqui-valenten Triethylamin unter Eiskühlung umgesetzt. Nach einer Stunde wäscht man die Reaktionslösung mit verdünnter Salzsäure (2 mal) und Wasser und engt die organische Phase nach dem Trocknen über Magnesiumsulfat ein. Das Produkt wird durch Kristalli-sation aus Ethylacetat/Petrolether und durch Chromatographie an Kieselgel (Toluol/Aceton 4:1) gereinigt. Man erhält 3-p-(5-Oxo-1,2,4-oxadiazolin-3-yl)-phenyl-5-[p-(2-benzyloxy-carbonyl-2-N-propylsulfonyl-amino-ethyl)-phenoxy]-methyl-oxazolidin-2-on; FAB-MS: m/e 635 (M+H⁺).

Analog erhält man durch Umsetzung von 3-p-(5-Oxo-1,2,4-oxadiazolin-3-yl)-phenyl-5-[p-(2-benzyloxycarbonyl-2-amino-ethyl)-phenoxy]-methyl-oxazolidin-2-on
mit Ethansulfonsäurechlorid das
3-p-(5-Oxo-1,2,4-oxadiazolin-3-yl)-phenyl-5-[p-(2-benzyloxycarbonyl-2-N-ethylsulfonyl-amino-ethyl)-phenoxy]-methyl-oxazolidin-2-on;
mit Toluolsulfonsäurechlorid das
3-p-(5-Oxo-1,2,4-oxadiazolin-3-yl)-phenyl-5-[p-(2-benzyloxycarbonyl-2-N-toluolsulfonyl-amino-ethyl)-phenoxy]-methyl-oxazolidin-2-on;
mit Benzylsulfonsäurechlorid das
3-p-(5-Oxo-1,2,4-oxadiazolin-3-yl)-phenyl-5-[p-(2-benzyloxycarbonyl-2-N-benzylsulfonyl-amino-ethyl)-phenoxy]-methyl-oxazolidin-2-on;
mit Hexansulfonsäurechlorid das
3-p-(5-Oxo-1,2,4-oxadiazolin-3-yl)-phenyl-5-[p-(2-benzyloxycarbonyl-2-N-hexylsulfonyl-amino-ethyl)-phenoxy]-methyl-oxazolidin-2-on;
mit Pentansulfonsäurechlorid das
3-p-(5-Oxo-1,2,4-oxadiazolin-3-yl)-phenyl-5-[p-(2-benzyloxycarbonyl-2-N-pentylsulfonyl-amino-ethyl)-phenoxy]-methyl-oxazolidin-2-on;
mit Hexadekansulfonsäurechlorid das
3-p-(5-Oxo-1,2,4-oxadiazolin-3-yl)-phenyl-5-[p-(2-benzyloxycarbonyl-2-N-hexadekanylsulfonyl-amino-ethyl)-phenoxy]-methyl-oxazolidin-2-on;
mit Butansäurechlorid das
3-p-(5-Oxo-1,2,4-oxadiazolin-3-yl)-phenyl-5-[p-(2-benzyloxycarbonyl-2-N-butanoyl-amino-ethyl)-phenoxy]-methyl-oxazolidin-2-on;
mit Pentansäurechlorid das
3-p-(5-Oxo-1,2,4-oxadiazolin-3-yl)-phenyl-5-[p-(2-benzyloxycarbonyl-2-N-pentanoyl-amino-ethyl)-phenoxy]-methyl-oxazolidin-2-on;
mit Hexansäurechlorid das
3-p-(5-Oxo-1,2,4-oxadiazolin-3-yl)-phenyl-5-[p-(2-benzyloxycarbonyl-2-N-hexanoyl-amino-ethyl)-phenoxy]-methyl-oxazolidin-2-on;
mit Benzoesäurechlorid das
3-p-(5-Oxo-1,2,4-oxadiazolin-3-yl)-phenyl-5-[p-(2-benzyloxycarbonyl-2-N-benzoyl-amino-ethyl)-phenoxy]-methyl-oxazolidin-2-on;
mit 1-Naphthalinsäurechlorid das
3-p-(5-Oxo-1,2,4-oxadiazolin-3-yl)-phenyl-5-[p-(2-benzyloxycarbonyl-2-N-(1-naphthoyl-amino)-ethyl)-phenoxy]-methyl-oxazolidin-2-on;
mit 2-Naphthalinsäurechlorid das
3-p-(5-Oxo-1,2,4-oxadiazolin-3-yl)-phenyl-5-[p-(2-benzyloxycarbonyl-2-N-(2-naphthoyl-amino)-ethyl)-phenoxy]-methyl-oxazolidin-2-on.

### Beispiel 17

Zur Spaltung des Benzylesters und des Oxadiazolinringes werden 0,4 g 3-p-(5-Oxo-1,2,4-oxadiazolin-3-yl)-phenyl-5-[p-(2-benzyloxy-carbonyl-2-N-propylsulfonyl-amino-ethyl)-phenoxy]-methyl-oxazolidin-2-on in 10 ml Dichlormethan gelöst und nach Zusatz von 1 ml Essigsäure, 0,5 ml Wasser und 5 ml Methanol sowie 0,1 g Palladium/Aktivkohle (10%) bei Raumtemperatur mit Wasserstoff behandelt. Nach 30 Minuten filtriert man den Katalysator ab, engt die Reaktionslösung ein und erhält das 3-p-Amidino-phenyl-5-[p-(2-carboxy-2-N-propylsulfonyl-amino-ethyl)-phenoxy]-methyl-oxazolidin-2-on, F. 200° (Zers.).

Analog erhält man durch Hydrierung der Produkte aus Beispiel 16
3-p-Amidino-phenyl-5-[p-(2-carboxy-2-N-ethylsulfonyl-amino-ethyl)-phenoxy]-methyl-oxazolidin-2-on, F. 212° (Zers.);
3-p-Amidino-phenyl-5-[p-(2-carboxy-2-N-toluolsulfonyl-amino-ethyl)-phenoxy]-methyl-oxazolidin-2-on, F. 205° (Zers.);
3-p-Amidino-phenyl-5-[p-(2-carboxy-2-N-benzylsulfonyl-amino-ethyl)-phenoxy]-methyl-oxazolidin-2-on, F. 211° (Zers.);
3-p-Amidino-phenyl-5-[p-(2-carboxy-2-N-hexylsulfonyl-amino-ethyl)-phenoxy]-methyl-oxazolidin-2-on, F. 198° (Zers.);
3-p-Amidino-phenyl-5-[p-(2-carboxy-2-N-pentylsulfonyl-amino-ethyl)-phenoxy]-methyl-oxazolidin-2-on, F. 215° (Zers.);
3-p-Amidino-phenyl-5-[p-(2-carboxy-2-N-hexadekanylsulfonyl-amino-ethyl)-phenoxy]-methyl-oxazolidin-2-on, F. 220° (Zers.);
3-p-Amidino-phenyl-5-[p-(2-carboxy-2-N-butanoyl-amino-ethyl)-phenoxy]-methyl-oxazolidin-2-on, F. 190° (Zers.);
3-p-Amidino-phenyl-5-[p-(2-carboxy-2-N-pentanoyl-amino-ethyl)-phenoxy]-methyl-oxazolidin-2-on, F. 195° (Zers.);
3-p-Amidino-phenyl-5-[p-(2-carboxy-2-N-hexanoyl-amino-ethyl)-phenoxy]-methyl-oxazolidin-2-on, F. 188° (Zers.);
3-p-Amidino-phenyl-5-[p-(2-carboxy-2-N-benzoyl-amino-ethyl)-phenoxy]-methyl-oxazolidin-2-on, F. 204° (Zers.);
3-p-Amidino-phenyl-5-[p-(2-carboxy-2-N-(1-naphthoyl-amino)-ethyl)-phenoxy]-methyl-oxazolidin-2-on;
3-p-Amidino-phenyl-5-[p-(2-carboxy-2-N-(2-naphthoyl-amino)-ethyl)-phenoxy]-methyl-oxazolidin-2-on, F. 226° (Zers.).

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Oxazolidinon-Derivate der Formel I 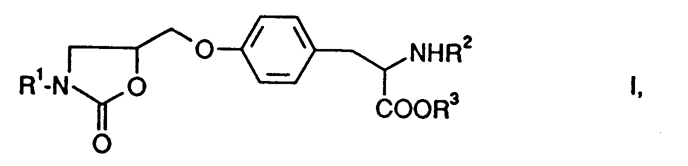 worin
R¹ 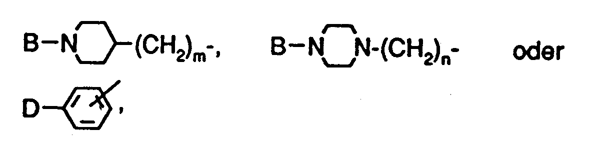
R² H, A, Ac, A-SO₂-, Ar-SO₂- oder eine konventionelle Aminoschutzgruppe,
R³ H, A, Cycloalkyl mit 3 bis 7 C-Atomen, Ar oder Ar-(CH₂)ₖ-,
A Alkyl mit 1 bis 16 C-Atomen,
B H, A oder H₂N-C(=NH)-,
D H₂N-CH₂-, H₂N-C(=NH)- oder H₂N-C(=NH)-NH-CH₂, wobei die primären Aminogruppen auch mit konventionellen Aminoschutzgruppen versehen sein können,
Ac Alkanoyl mit 1 bis 10 C-Atomen oder Aroyl mit 7 bis 11 C-Atomen,
Ar unsubstituiertes oder durch A, Cl, Br, I, OA, OH, NO₂, CN, NH₂, NHA und/oder NA₂ mono- oder disubstituiertes Phenyl oder Benzyl,
m 0, 1, 2, 3 oder 4,
n 2, 3 oder 4 und
k 1, 2, 3 oder 4,
bedeuten,
sowie deren physiologisch unbedenkliche Salze.

2. Enantiomere oder Diastereomere der Verbindungen der Formel I gemäß Anspruch 1.

3. (a) 3-p-Amidino-phenyl-5-[p-(2-amino-2-carboxy-ethyl)-phenoxy-methyl]-oxazolidin-2-on;
(b) 3-p-Amidino-phenyl-5-[p-(2-amino-2-methoxycarbonyl-ethyl)-phenoxy-methyl]-oxazolidin-2-on;
(c) 3-p-Amidino-phenyl-5-[p-(2-N-butylsulfonyl-amino-2-carboxy-ethyl)-phenoxy-methyl]-oxazolidin-2-on;
(d) 3-p-Amidinophenyl-5-[p-(2-N-p-toluolsulfonyl-amino-2-carboxy-ethyl)-phenoxy-methyl]-oxazolidin-2-on;
(e) 3-p-Amidino-phenyl-5-[p-(2-N-butylsulfonyl-amino-2-methoxy-carbonyl-ethyl)-phenoxy-methyl]-oxazolidin-2-on,
sowie die physiologisch unbedenklichen Säureadditionssalze der genannten Verbindungen.

4. Verfahren zur Herstellung von Verbindungen der Formel I gemäß Anspruch 1, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Verbindung der Formel II 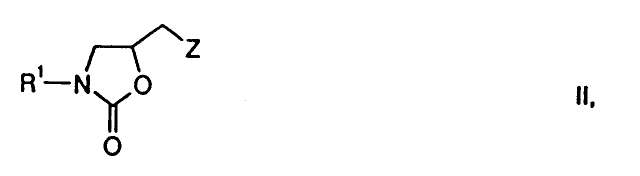 worin
R¹ die in Anspruch 1 angegebene Bedeutung besitzt
und
Z Cl, Br, I, OH oder eine reaktionsfähig veresterte OH-Gruppe bedeutet,
mit einer Verbindung der Formel III 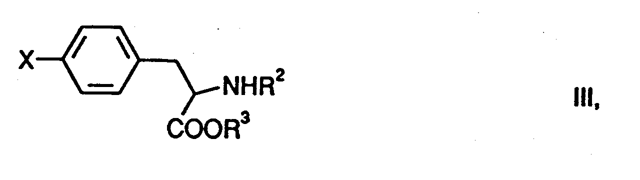 worin
R² und R³ die angegebenen Bedeutungen haben
und
X OH oder einen aus OH ableitbaren salzartigen Rest bedeutet,
umsetzt, oder daß man
(c) eine Verbindung der Formel IV 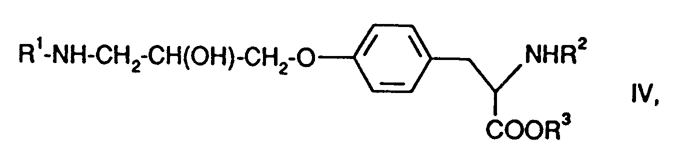 worin
R¹, R² und R³ die angegebenen Bedeutungen haben,
mit einem reaktionsfähigen Derivat der Kohlensäure umsetzt, oder daß man
(d) eine Verbindung der Formel V 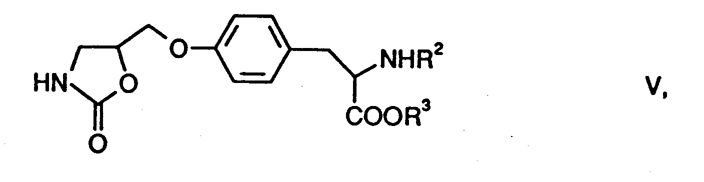 worin
R² und R³ die angegebenen Bedeutungen haben, mit einer Verbindung der Formel VI 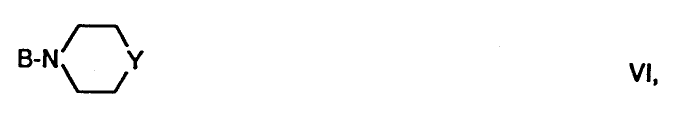 worin
B die angegebene Bedeutung hat
und
Y 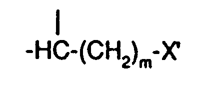 oder 〉N-(CH₂)ₙ-X',
worin m und n die bereits angegebenen Bedeutungen haben und
X' Cl, Br, I oder eine andere leicht verdrängbare Abgangsgruppe ist, bedeutet,
umsetzt, oder daß man
(e) zur Herstellung einer Guanidinomethylverbindung der Formel I (R¹ = einfach durch H₂N-C(=NH)-NH-CH₂-substituierter Phenylrest) eine Aminoverbindung entsprechend der Formel I, die jedoch anstelle des Restes R¹ eine Aminomethylphenylgruppe enthält, mit einem amidinierenden Mittel behandelt, oder daß man
(f) einen Rest R³ in einen anderen Rest R³ umwandelt, indem man einen Ester der Formel I verseift, oder eine Carbonsäure der Formel I verestert, oder daß man
(g) (einen) Rest(e) R¹ und/oder R² in (einen) andere(n) Rest(e) R¹ und/oder R² umwandelt, und/oder daß man
(h) eine Verbindung der Formel I durch Behandeln mit einer Säure oder Base in eines ihrer Salze überführt.

5. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

6. Pharmazeutische Zubereitung, gekennzeichnet durch einen Gehalt an mindestens einer Verbindung der Formel I, gemäß Anspruch 1, und/oder einem ihrer physiologisch unbedenklichen Salze.

7. Verwendung einer Verbindung der Formel I gemäß Anspruch 1 und/oder eines ihrer physiologisch unbedenklichen Salze zur Herstellung von Arzneimitteln.

## Claims

1. Oxazolidinone derivatives of the formula I 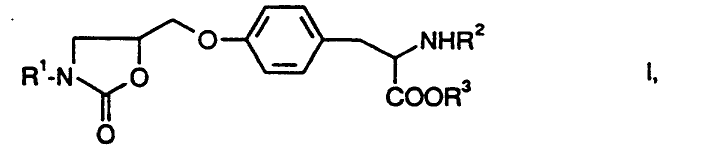 wherein
R¹ is 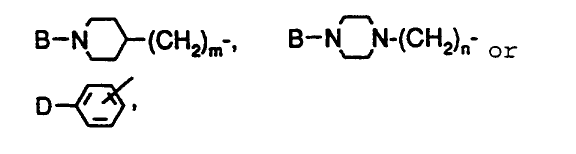
R² is H, A, Ac, A-SO₂-, Ar-SO₂- or a conventional amino protective group,
R³ is H, A, cycloalkyl having 3 to 7 C atoms, Ar or Ar-(CH₂)ₖ-,
A is alkyl having 1 to 16 C atoms,
B is H, A or H₂N-C(=NH)-,
D is H₂N-CH₂-, H₂N-C(=NH) - or H₂N-C(=NH)-NH-CH₂-, where the primary amino groups can also be provided with conventional amino protective groups,
Ac is alkanoyl having 1 to 10 C atoms or aroyl having 7 to 11 C atoms,
Ar is phenyl or benzyl which is unsubstituted, mono-or disubstituted by A, Cl, Br, I, OA, OH, NO₂, CN, NH₂, NHA and/or NA₂,
m is 0, 1, 2, 3 or 4,
n is 2, 3 or 4 and
k is 1, 2, 3 or 4,
and their physiologically acceptable salts.

2. Enantiomers or diastereomers of the compounds of the formula I according to Claim 1.

3. (a) 3-p-Amidinophenyl-5-[p-(2-amino-2-carboxyethyl)phenoxymethyl]oxazolidin-2-one;
(b) 3-p-amidinophenyl-5-[p-(2-amino-2-methoxycarbonylethyl)phenoxymethyl]-oxazolidin-2-one;
(c) 3-p-amidinophenyl-5-[p-(2-N-butylsulfonylamino-carboxyethyl)phenoxymethyl]oxazolidin-2-one;
(d) 3-p-amidinophenyl-5-[p-(2-N-p-toluenesulfonylamino-N-carboxyethyl)phenoxymethyl]oxazolidin-2-one;
(e) 3-p-amidinophenyl-5-[p-(2-N-butylsulfonylamino-2-methoxycarbonylethyl)phenoxymethyl]-oxazolidin-2-one,
and the physiologically acceptable acid addition salts of the compounds mentioned.

4. Process for the preparation of compounds of the formula I according to Claim 1, characterized in that
(a) a compound of the formula I is liberated from one of its functional derivatives by treating with a solvolysing or hydrogenolysing agent, or in that
(b) a compound of the formula II 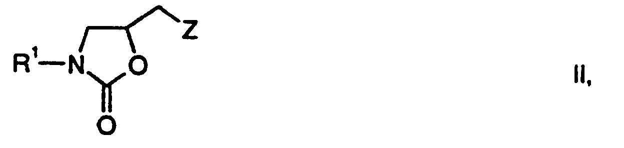 wherein
R¹ has the meaning given in Claim 1
and
Z is Cl, Br, I, OH or a reactive esterified OH group,
is reacted with a compound of the formula III 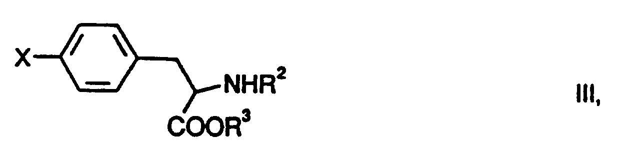 wherein
R² and R³ have the meanings indicated
and
X is OH or a salt-like radical derivable from OH,
or in that
(c) a compound of the formula IV 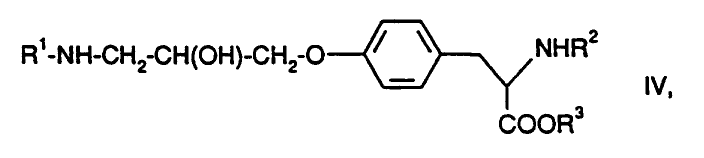 wherein
R¹, R² and R³ have the meanings indicated,
is reacted with a reactive derivative of carbonic acid, or in that
(d) a compound of the formula V 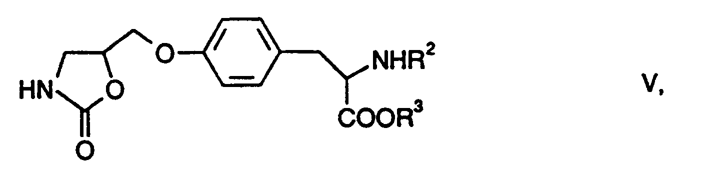 wherein
R² and R³ have the meanings indicated, is reacted with a compound of the formula VI 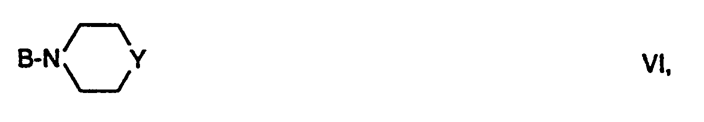 wherein
B has the meaning indicated
and
Y is 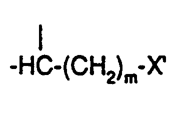 or 〉N-(CH₂)ₙ-X',
wherein
m and n have the meanings already indicated and
X' is Cl, Br, I or another, easily nucleophilically displaceable leaving group,
or in that
(e) for the preparation of a guanidinomethyl compound of the formula I (R¹ = a phenyl radical monosubstituted by H₂N-C(=NH)-NH-CH₂-), an amino compound corresponding to the formula I, but which instead of the radical R¹ contains an aminomethylphenyl group, is treated with an amidinating agent, or in that
(f) a radical R³ is converted into another radical R³ by hydrolysing an ester of the formula I, or a carboxylic acid of the formula I is esterified, or in that
(g) (a) radical(s) R¹ and/or R² is (are) converted into (an)other radical(s) R¹ and/or R², and/or in that
(h) a compound of the formula I is converted into one of its salts by treating with an acid or base.

5. Process for the production of a pharmaceutical preparation, characterized in that a compound of the formula I according to Claim 1 and/or one of its physiologically acceptable salts is brought into a suitable administration form together with at least one solid, liquid or semi-liquid excipient or auxiliary.

6. Pharmaceutical preparation, characterized in that it contains at least one compound of the formula I according to Claim 1, and/or one of its physiologically acceptable salts.

7. Use of a compound of the formula I according to Claim 1 and/or of one of its physiologically acceptable salts for the production of medicaments.

## Revendications

1. Dérivés de l'oxazolidinone de la formule I : 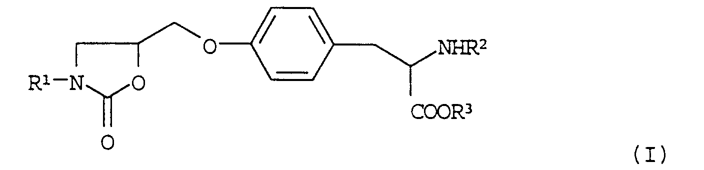 dans laquelle :
R¹ est 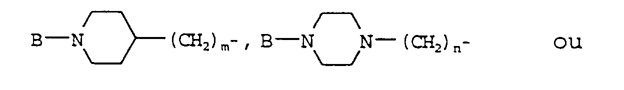 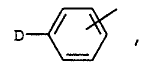
R² est H, A, Ac, A-SO₂-, Ar-SO₂- ou un groupe amino protecteur classique,
R³ est H, A, un cycloalkyle ayant de 3 à 7 atomes de carbone, Ar ou Ar-(CH₂)ₖ-,
A est un alkyle ayant de 1 à 16 atomes de carbone,
B est H, A ou H₂N-C(=NH)-,
D est H₂N-CH₂-, H₂N-C(=NH)- ou H₂N-C(=NH)-NH-CH₂-, les groupes amino primaires pouvant être également munis de groupes amino protecteurs classiques,
Ac est un alcanoyle ayant de 1 à 10 atomes de carbone ou un aroyle ayant de 7 à 11 atomes de carbone,
Ar est un phényle ou un benzyle non substitué ou bien mono ou disubstitué par A, Cl, Br, I, OA, OH, NO₂, CN, NH₂, NHA et/ou NA₂,
m est égal à 0, 1, 2, 3 ou 4,
n est égal à 2, 3 ou 4, et
k est égal à 1, 2, 3 ou 4,
ainsi que leurs sels physiologiquement acceptables.

2. Enantiomères ou diastéréomères des composés de la formule I selon la revendication 1.

3. (a) 3-p-amidinophényl-5-[p-(2-amino-2-
carboxyéthyl)-phénoxyméthyl]-oxazolidin-2-one ;
(b) 3-p-amidinophényl-5-[p-(2-amino-2-méthoxycarbonyléthyl)-phénoxyméthyl]-oxazolidin-2-one ;
(c) 3-p-amidinophényl-5-[p-(2-N-butylsulfonyl-amino-2-carboxyéthyl)-phénoxyméthyl]oxazolidin-2-one ;
(d) 3-p-amidinophényl-5-[p-(2-N-p-toluènesulfonyl-amino-2-carboxyéthyl)-phénoxyméthyl]oxazolidin-2-one ;
(e) 3-p-amidinophényl-5-[p-(2-N-butylsulfonyl-amino-2-méthoxycarbonyléthyl)-phénoxyméthyl]-oxazolidin-2-one ;
ainsi que les sels d'addition acide physiologiquement acceptables des composés précités.

4. Procédé de préparation des composés de la formule I selon la revendication 1, caractérisé en ce que :
(a) on libère un composé de la formule I à partir de ses dérivés fonctionnels en les traitant avec un agent solvolysant ou hydrogénolysant,
ou bien en ce que
(b) on fait réagir un composé de la formule II : 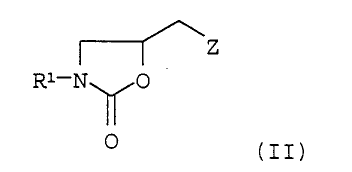 dans laquelle :
R¹ a la signification indiquée dans la revendication 1
et
Z est Cl, Br, I, OH ou un groupe OH estérifié réactif,
avec un composé de la formule III : 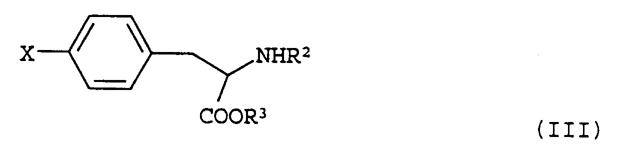 dans laquelle :
R² et R³ ont les significations indiquées
et
X est OH ou un reste de type sel dérivable de OH,
ou bien en ce que
(c) on fait réagir un composé de la formule IV : 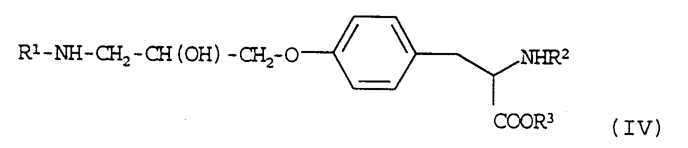 dans laquelle :
R¹, R² et R³ ont les significations indiquées,
avec un dérivé réactif de l'anhydride carbonique,
ou bien en ce que
(d) on fait réagir un composé de la formule V : 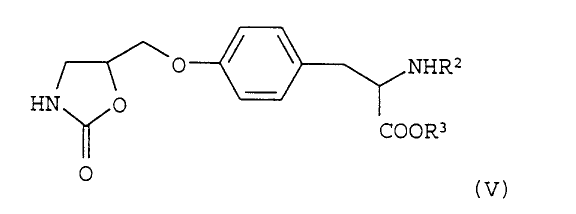 dans laquelle :
R² et R³ ont les significations indiquées,
avec un composé de la formule VI : 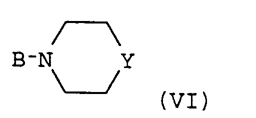 dans laquelle :
B a la signification indiquée
et
Y est 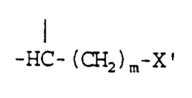 ou 〉N-(CH₂)ₙ-X' ,
formules dans lesquelles m et n ont les significations déjà indiquées et
X' est Cl, Br, I ou un groupe de départ facilement déplaçable,
ou bien en ce que
(e) pour préparer un composé guanidinométhyle de la formule I (R¹ = reste phényle simplement substitué par H₂N-C(=NH)-NH-CH₂), on traite un composé amino correspondant à la formule I mais contenant un groupe aminométhylphényle à la place du reste R¹ avec un agent amidinant,
ou bien en ce que
(f) on convertit un reste R³ en un autre reste R³ en saponifiant un ester de la formule I ou en estérifiant un acide carboxylique de la formule I,
ou bien en ce que
(g) on convertit un ou plusieurs restes R¹ et/ou R² en un ou plusieurs autres restes R¹ et/ou R²,
et/ou en ce que
(h) on transforme un composé de la formule I en un de ses sels en le traitant avec un acide ou une base.

5. Procédé de fabrication d'une préparation pharmaceutique, caractérisé en ce que l'on conditionne un composé de la formule I selon la revendication 1 et/ou un de ses sels physiologiquement acceptables ensemble avec au moins un véhicule ou un adjuvant solide, liquide ou semi-liquide pour obtenir une présentation appropriée.

6. Préparation pharmaceutique, caractérisé en ce qu'elle contient au moins un composé de la formule I selon la revendication 1 et/ou un de ses sels physiologiquement acceptables.

7. Utilisation d'un composé de la formule I selon la revendication 1 et/ou d'un de ses sels physiologiquement acceptables pour préparer des médicaments.
